# EUROPEAN PATENT APPLICATION

(11) **EP 4 610 254 A1**
(43) Date of publication of application: **03.09.2025**
(21) Application number: 23882574.9
(22) Date of filing: 23.10.2023
(51) Int. Cl.: C07D 221/18, C07D 401/04, C08G 59/40

(54) **COMPOUND, COMPOSITION, CURED PRODUCT, METHOD FOR PRODUCING CURED PRODUCT, AND METHOD FOR PRODUCING ELECTRONIC COMPONENT**

(30) Priority: 26.10.2022 JP 2022171826
(71) Applicant: ADEKA CORPORATION, Arakawa-ku Tokyo 116-8554 (JP)
(72) Inventor: MURAMATSU, Yousuke, Tokyo 116-8554 (JP); KANEHARA, Yukiko, Tokyo 116-8554 (JP); SAITO, Hiromasa, Tokyo 116-8554 (JP)
(74) Representative: Forstmeyer, Dietmar
(86) International application number: PCT/JP2023/038145
(87) International publication number: WO 2024/090369

(57) **Abstract**

Provided is a compound represented by a general formula (I) below: 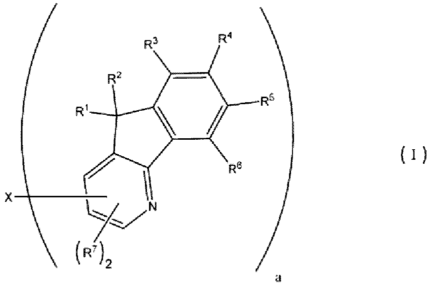 where X represents a hydrogen atom, an optionally substituted ring group having 2 to 20 ring-constituting carbon atoms, an optionally substituted C1-20 chain hydrocarbon group, or another group; R¹ to R⁷ each represent a hydrogen atom, a reactive group selected from <Group A>, an optionally substituted C1-20 alkyl group, a an optionally substituted C6-20 aromatic ring group, or another group; <Group A> consists of a carbon-carbon unsaturated bond-containing group, a cyclic ether group, a hydroxyl group, a mercapto group, an amino group, a halogen atom, and a cyano group; a represents an integer of 1 to 10; R¹ and R² may be bonded together to form a ring, R³ and R⁴ may be bonded together to form a ring, R⁴ and R⁵ are optionally bonded together to form a ring, R⁵ and R⁶ are optionally bonded together to form a ring, and a plurality of R⁷ are optionally bonded together to form a ring; provided that the compound has one or more reactive groups selected from <Group A> in a molecule thereof. See the specification for definitions of others.

## Description

### Technical Field

The present invention relates to a compound having a specific skeleton.

### Background Art

Conventionally, it has been known that compounds with a skeletal structure composed of a six-membered ring and a five-membered ring fused together have heat resistance.

Non-Patent Literature 1 discloses the structure of 11-aryl-10,12-dihydrodiindino[1,2-b',2',1'-e]pyridine and a production method thereof.

Patent Literature 1 describes a polymerized product having a structure containing an indolocarbazole unit in a side chain, and a polymer containing this polymerized product, and further states that a resist underlayer film containing this polymer does not cause intermixing with a resist layer, and has high dry etching resistance and high heat resistance, and that the amount of sublimates therefrom is small.

Patent Literature 2 states that a compound with a predetermined skeleton containing two or more indole structures is excellent in heat resistance and solvent resistance.

### Citation List

### Patent Literature

Patent Literature 1: US 2018/0356732A1
Patent Literature 2: WO 2022/131346

### Non-Patent Literature

Non-Patent Literature 1: J. Chem. Res. (2016), Vol. 40, p. 428-435 (Abudullah M. Asrl et al.)

### Summary of Invention

### Problems to be Solved by the Invention

An object of the present invention is to provide a compound that gives a cured product excellent in heat resistance and solvent resistance, and a composition containing the same.

### Means to Solve the Problems

As a result of in-depth research, the inventors of the present invention have found that a compound having a specific skeleton and also having one or more reactive groups in its molecule and a composition containing the same give a cured product excellent in heat resistance and solvent resistance, and thus accomplished the present invention.

Specifically, the present invention provides [1] to [13] below.
[1] A compound represented by a general formula (I) below: 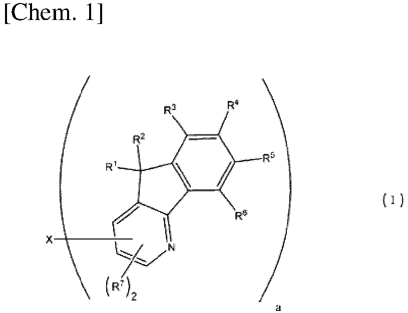
   where X represents a hydrogen atom, an optionally substituted ring group having 2 to 20 ring-constituting carbon atoms, an optionally substituted chain hydrocarbon group having 1 to 20 carbon atoms, or a group obtained by replacing one or more methylene groups in the chain hydrocarbon group with a divalent group selected from <Group B> below;
   R¹, R², R³, R⁴, R⁵, R⁶, and R⁷ (hereinafter also referred to as "R¹ to R⁷") each independently represent a hydrogen atom, a reactive group selected from <Group A> below, a nitro group, an optionally substituted alkyl group having 1 to 20 carbon atoms, an optionally substituted aromatic ring group having 6 to 20 carbon atoms, an optionally substituted heterocyclic group having 2 to 20 carbon atoms, a group obtained by replacing one or more methylene groups in the alkyl group having 1 to 20 carbon atoms with a divalent group selected from <Group B> below, a group obtained by replacing one or more methylene groups in the aromatic ring group having 6 to 20 carbon atoms with a divalent group selected from <Group B> below, or a group obtained by replacing one or more methylene groups in the heterocyclic group having 2 to 20 carbon atoms with a divalent group selected from <Group B> below;
   <Group A> consists of a carbon-carbon unsaturated bond-containing group, a cyclic ether group, a hydroxyl group, a mercapto group, an amino group, a halogen atom, and a cyano group,
   <Group B> consists of -O-, -CO-, -COO-, -OCO-, -NR¹¹- , -NR¹¹CO-, and -S-, where R¹¹ represents a hydrogen atom or a hydrocarbon group;
   *a* represents an integer of 1 to 10,
   when X is a hydrogen atom, *a* is 1, and
   when *a* is 2 or more, a plurality of R¹ are the same or different, a plurality of R² are the same or different, a plurality of R³ are the same or different, a plurality of R⁴ are the same or different, a plurality of R⁵ are the same or different, a plurality of R⁶ are the same or different, and a plurality of R⁷ are the same or different;
   two R⁷ in one repeating unit are the same or different;
   R¹ and R² are optionally bonded together to form a ring,
   R³ and R⁴ are optionally bonded together to form a ring,
   R⁴ and R⁵ are optionally bonded together to form a ring,
   R⁵ and R⁶ are optionally bonded together to form a ring,
   a plurality of R⁷ are optionally bonded together to form a ring;
   provided that the compound has one or more reactive groups selected from <Group A> in a molecule thereof.
[2] The compound as set forth in claim 1, represented by a general formula (IIα), (IIβ), or (IIγ) below: 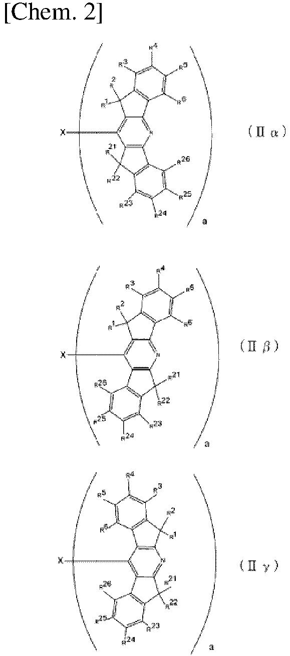
   where X, *a,* R¹, R², R³, R⁴, R⁵, and R⁶ are as in the general formula (I);
   R²¹, R²², R²³, R²⁴, R²⁵, and R²⁶ (hereinafter also referred to as "R²¹ to R²⁶") each independently represent a hydrogen atom, a reactive group selected from <Group A> above, a nitro group, an optionally substituted alkyl group having 1 to 20 carbon atoms, an optionally substituted aromatic ring group having 6 to 20 carbon atoms, an optionally substituted heterocyclic group having 2 to 20 carbon atoms, a group obtained by replacing one or more methylene groups in the alkyl group having 1 to 20 carbon atoms with a divalent group selected from <Group B> above, a group obtained by replacing one or more methylene groups in the aromatic ring group having 6 to 20 carbon atoms with a divalent group selected from <Group B> above, or a group obtained by replacing one or more methylene groups in the heterocyclic group having 2 to 20 carbon atoms with a divalent group selected from <Group B> above;
   R²¹ and R²² are optionally bonded together to form a ring,
   R²³ and R²⁴ are optionally bonded together to form a ring,
   R²⁴ and R²⁵ are optionally bonded together to form a ring, and
   R²⁵ and R²⁶ are optionally bonded together to form a ring.
[3] The compound as set forth in [1] or [2], wherein X is an optionally substituted aromatic ring group having 6 to 20 carbon atoms or an optionally substituted heterocyclic group having 2 to 20 carbon atoms.
[4] The compound as set forth in [1], comprising, in the molecule, one or more carbon-carbon unsaturated bond-containing groups as the reactive group.
[5] The compound as set forth in [2], comprising, in the molecule, one or more carbon-carbon unsaturated bond-containing groups as the reactive group.
[6] The compound as set forth in any one of [1] to [5], wherein the number of the reactive groups selected from <Group A> above is 2 or more in the molecule.
[7] The compound as set forth in any one of [1] to [6], wherein X has an optionally substituted fused ring, or the number of the reactive groups in the molecule is three or more.
[8] A composition comprising the compound as set forth in any one of [1] to [7].
[9] The composition as set forth in [8], being for an electronic component.
[10] A cured product from the composition as set forth in [8].
[11] A method for producing a cured product, comprising the step of curing the composition as set forth in [8].
[12] A method for producing an electronic component, comprising the step of curing the composition as set forth in [8] and then the step of removing the cured product.
[13] A compound represented by a general formula (III) below: 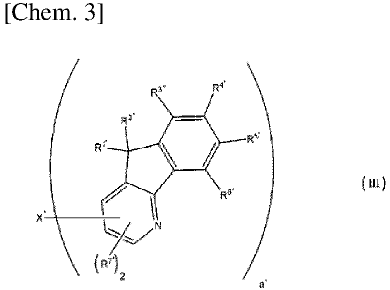
   where X' represents a hydrogen atom, an optionally substituted ring group having 2 to 20 ring-constituting carbon atoms, an optionally substituted chain hydrocarbon group having 1 to 20 carbon atoms, or a group obtained by replacing one or more methylene groups in the chain hydrocarbon group with a divalent group selected from <Group B> below;
   R¹', R²', R³', R⁴', R⁵', R⁶', and R⁷' each independently represent a hydrogen atom, a reactive group selected from <Group A> below, a nitro group, an optionally substituted alkyl group having 1 to 20 carbon atoms, an optionally substituted aromatic ring group having 6 to 20 carbon atoms, an optionally substituted heterocyclic group having 2 to 20 carbon atoms, a group obtained by replacing one or more methylene groups in the alkyl group having 1 to 20 carbon atoms with a divalent group selected from <Group B> below, a group obtained by replacing one or more methylene groups in the aromatic ring group having 6 to 20 carbon atoms with a divalent group selected from <Group B> below, or a group obtained by replacing one or more methylene groups in the heterocyclic group having 2 to 20 carbon atoms with a divalent group selected from <Group B> below;
   <Group A> consists of a carbon-carbon unsaturated bond-containing group, a cyclic ether group, a hydroxyl group, a mercapto group, an amino group, a halogen atom, and a cyano group,
   <Group B> consists of -O-, -CO-, -COO-, -OCO-, -NR¹¹'-, -NR¹¹'CO-, and -S-, where R¹¹' represents a hydrogen atom or a hydrocarbon group;
   *a*' represents an integer of 1 to 10,
   when X' is a hydrogen atom, *a* is 1, and
   when *a*' is 2 or more, a plurality of R¹' are the same or different, a plurality of R²' are the same or different, a plurality of R³' are the same or different, a plurality of R⁴' are the same or different, a plurality of R⁵' are the same or different, a plurality of R⁶' are the same or different, and a plurality of R⁷' are be the same or different;
   two R⁷' in one repeating unit are the same or different;
   R¹' and R²' are optionally bonded together to form a ring,
   R³' and R⁴' are optionally bonded together to form a ring,
   R⁴' and R⁵' are optionally bonded together to form a ring,
   R⁵' and R⁶' are optionally bonded together to form a ring,
   a plurality of R⁷' are optionally bonded together to form a ring;
   provided that the compound has one or more phenolic hydroxyl groups in a molecule thereof.

### Advantageous Effects of Invention

The compound of the present invention, which is represented by the general formula (I), and the composition containing the same give a cured product excellent in heat resistance and solvent resistance. The cured product of the present invention, which is obtained by using the above-described composition, is also excellent in heat resistance and solvent resistance. The method of the present invention, which is for producing an electronic component, involves use of the composition suitable for electronic components that require high heat resistance and high solvent resistance, so that high-grade electronic components can be produced by the method. Furthermore, the compound represented by a general formula (III) is a precursor of the compound of the present invention, and by using this compound, the compound of the present invention can be successfully produced.

### Description of Embodiment

### A. Compound Represented by General Formula (I)

First, the compound represented by the general formula (I) (hereinafter also referred to as a "compound I") will be described. The compound I is a compound represented by the general formula (I) above and having a specific skeleton, and is characterized by having a predetermined skeleton and having, in its molecule, one or more reactive groups selected from <Group A> above (hereinafter also simply referred to as "reactive groups"). Preferred examples of the compound I are compounds represented by the general formulas (IIα), (IIβ), and (IIγ) (hereinafter also referred to as a "compound IIα", a "compound IIβ", and a "compound IIγ" respectively, and these three compounds are also collectively referred to as "compounds IIα to IIγ").

The expression "two R⁷ in one repeating unit" means two R⁷ in "(R⁷)2" in the formula (I).

Among the above-mentioned reactive groups, the carbon-carbon unsaturated bond-containing group refers to a group having a carbon-carbon unsaturated bond, and is preferably a group having a carbon-carbon unsaturated bond at the terminal. Preferred examples of the group having a carbon-carbon unsaturated bond at the terminal, as the carbon-carbon unsaturated bond-containing group, include groups having a carbon-carbon triple bond at the terminal such as an ethynyl group, a propargyl group, a propargyloxy group, a monopropargylamino group, and a dipropargylamino group (di-2-propynylamino group), and groups having a carbon-carbon double bond at the terminal such as a vinyl group, an allyl group, an acrylic group, an acryloyloxy group, a methacrylic group, and a methacryloxy group. The carbon-carbon unsaturated bond-containing group as the reactive group is preferably a group having a carbon-carbon triple bond at the terminal, and more preferably an ethynyl group, a propargyl group, a propargyloxy group, or a dipropargylamino group. When the carbon-carbon unsaturated bond-containing group is a group as mentioned above, a cured product excellent in heat resistance and solvent resistance can be easily obtained.

Among the above-mentioned reactive groups, the cyclic ether group refers to a group having a cyclic ether, preferably a group having a cyclic ether at the terminal, more preferably an epoxy group or an oxetanyl group, and even more preferably an epoxy group such as a glycidyl ether group, a β-glycidyl ether group, or an alicyclic epoxy group. When the cyclic ether group is a group as mentioned above, a cured product having excellent heat resistance can be obtained.

The alkyl group having 1 to 20 carbon atoms and represented by R¹ to R⁷ in the formula (I) and R²¹ to R²⁶ in the formulas (IIα) to (IIγ) may be linear or branched or may have an alicyclic ring, and the alkyl group may be an arylalkyl group. Examples of the linear alkyl group include methyl, ethyl, propyl, butyl, iso-amyl, tert-amyl, hexyl, heptyl, and octyl. Examples of the branched alkyl group include iso-propyl, sec-butyl, tert-butyl, iso-butyl, iso-pentyl, tert-pentyl, 2-hexyl, 3-hexyl, 2-heptyl, 3-heptyl, iso-heptyl, tert-heptyl, iso-octyl, tert-octyl, 2-ethylhexyl, nonyl, isononyl, decyl, dodecyl, tridecyl, tetradecyl, pentadecyl, hexadecyl, heptadecyl, and octadecyl.

Examples of those having an alicyclic ring include: cycloalkyl groups such as cyclopentyl and cyclohexyl; cycloalkylalkyl groups such as cyclohexylmethyl; and alkyl groups obtained by replacing a hydrogen atom in an alkyl group with a heterocyclic ring.

In a case in which an alkyl group other than the arylalkyl group is represented by any of R¹ to R⁷ and R²¹ to R²⁶, the number of carbon atoms in the alkyl group is preferably 1 to 15, more preferably 1 to 10, and even more preferably 1 to 5, in view of heat resistance and solvent resistance.

The above-mentioned arylalkyl group means a group obtained by replacing one or more hydrogen atoms in the alkyl group with an aryl group. Examples of the arylalkyl group include benzyl, fluorenyl, indenyl, 9-fluorenylmethyl, α-methylbenzyl, α,α-dimethylbenzyl, phenylethyl, and naphthylpropyl groups. In view of heat resistance and solvent resistance, the number of carbon atoms in the arylalkyl group represented by R¹ to R⁷ and R²¹ to R²⁶ is preferably 7 to 20, more preferably 7 to 15, and even more preferably 7 to 10.

The aromatic ring group having 6 to 20 carbon atoms represented by R¹ to R⁷ and R²¹ to R²⁶ is a hydrocarbon group including an aromatic hydrocarbon ring and having a bonding site on the aromatic hydrocarbon ring. The aromatic ring group may have an aliphatic hydrocarbon group or a heterocyclic ring, but preferably include no heterocyclic ring. If an aliphatic hydrocarbon group or heterocyclic ring is contained, the number of carbon atoms including that in the aliphatic hydrocarbon group or heterocyclic ring is 20 or less.

Examples of the aromatic ring group include an aryl group having 6 to 20 carbon atoms.

Examples of the aryl group having 6 to 20 carbon atoms include an aryl group having a monocyclic structure, a fused ring structure, or a structure composed of two or more aromatic hydrocarbon rings linked together.

Examples of the aryl group having 6 to 20 carbon atoms and having a monocyclic structure include phenyl, tolyl, xylyl, ethylphenyl, and 2,4,6-trimethylphenyl.

Examples of the aryl group having 6 to 20 carbon atoms and having a fused ring structure include naphthyl, anthracenyl, phenanthryl, pyrenyl, fluorenyl, and indenofluorenyl.

For the aryl group having two or more aromatic hydrocarbon rings linked together, examples of the linking group that links the aromatic hydrocarbon rings include a single bond, a sulfide group (-S-), and a carbonyl group.

Examples of the aryl group having two or more aromatic hydrocarbon rings linked together include biphenyl, diphenylsulfide, benzoylphenyl, a group having a monocyclic ring and a fused ring linked together by the linking group as mentioned above, and a group having fused rings linked together by the linking group as mentioned above.

In view of heat resistance and solvent resistance, the number of carbon atoms in the aromatic ring group represented by R¹ to R⁷ and R²¹ to R²⁶ is preferably 6 to 15, and more preferably 6 to 10.

Examples of the heterocyclic group having 2 to 20 carbon atoms represented by R¹ to R⁷ and R²¹ to R²⁶ include a group obtained by removing one hydrogen atom from a "heterocyclic compound" (hereinafter also referred to as a "heterocyclic group I") and a group obtained by replacing a hydrogen atom in the heterocyclic group I with a hydrocarbon group (hereinafter also referred to as a "heterocyclic group II"). Note that an epoxy group is not encompassed in the heterocyclic group because it is encompassed in the reactive group. The heterocyclic group may have a monocyclic structure or a fused ring structure. The heterocyclic group having 2 to 20 carbon atoms and having a fused ring structure may be, for example, a heterocyclic ring-containing fused ring group having 3 to 20 carbon atoms and having a structure composed of a heterocyclic ring and a heterocyclic or hydrocarbon ring fused together. Specific examples of the heterocyclic group I include pyridyl, quinolyl, thiazolyl, tetrahydrofuranyl, dioxolanyl, tetrahydropyranyl, methylthiophenyl, hexylthiophenyl, benzothiophenyl, pyrrolyl, pyrrolidinyl, imidazolyl, imidazolidinyl, imidazolinyl, pyrazolyl, pyrazolidinyl, piperidinyl, piperazinyl, pyrimidinyl, furyl, thienyl, benzoxazol-2-yl, thiazolyl, isothiazolyl, oxazolyl, isoxazolyl, and morpholinyl.

For the heterocyclic group II, examples of the hydrocarbon group replacing the hydrogen atom in the heterocyclic group I include those listed as a hydrocarbon group represented by R¹¹, which will be described later. The heterocyclic group II has a bonding site on the heterocyclic group I. In the case of the heterocyclic group II, the number of carbon atoms including that in the hydrocarbon group is "2 to 20".

In view of heat resistance and solvent resistance, the number of carbon atoms in the heterocyclic group represented by R¹ to R⁷ and R²¹ to R²⁶ is more preferably 2 to 15, even more preferably 2 to 10, and still more preferably 2 to 5.

Herein, the "group obtained by replacing one or more methylene groups in the alkyl group having 1 to 20 carbon atoms with a divalent group selected from <Group B>" is also referred to as an "alkyl group having 1 to 20 carbon atoms with replacement of methylene" hereinafter.

Similarly, the "group obtained by replacing one or more methylene groups in the aromatic ring group having 6 to 20 carbon atoms with a divalent group selected from <Group B>" is also referred to as a "aromatic ring group having 6 to 20 carbon atoms with replacement of methylene".

Similarly, the "group obtained by replacing one or more methylene groups in the heterocyclic group having 2 to 20 carbon atoms with a divalent group selected from <Group B>" is also referred to as a "heterocyclic group with replacement of methylene having 2 to 20 carbon atoms with replacement of methylene".

Herein, the number of carbon atoms of the alkyl group with replacement of methylene, the aromatic ring group with replacement of methylene, and the heterocyclic group with replacement of methylene, and a chain hydrocarbon group with replacement of methylene, which will be described later, is within the range in which a group with a predetermined number of carbon atoms before replacement of methylene is subjected to replacement of methylene. For example, herein, the number of carbon atoms of a group obtained by replacing one methylene group (-CH₂-) in an alkyl group having 20 carbon atoms with a divalent group "-O-" (e.g., an alkoxy group) is 19. The numbers of carbon atoms of the alkyl group with replacement of methylene, the aromatic ring group with replacement of methylene, and the heterocyclic group with replacement of methylene are in the range of 1 to 20, 6 to 20, and 2 to 20, respectively, when any group in Group B is selected, and the number of carbon atoms is preferably 15 or less, more preferably 10 or less, and even more preferably 5 or less.

Furthermore, the various groups with replacement of methylene mentioned above do not have a structure in which a plurality of the above-mentioned divalent groups are adjacent to each other.

R¹ and R², R³ and R⁴, R⁴ and R⁵, R⁵ and R⁶, or a plurality of R⁷ in the formula (I) may be bonded together to form a ring, and R²¹ and R²², R²³ and R²⁴, R²⁴ and R²⁵, or R²⁵ and R²⁶ in the formulas (IIα) to (IIγ) may be bonded together to form a ring. For these cases, examples of the ring include a hydrocarbon ring and a heterocyclic ring. Examples of the hydrocarbon ring include: monocyclic rings such as a cyclopetane ring, a cyclohexane ring, a cyclopentene ring, and a benzene ring; fused rings such as an indane ring, a fluorene ring, a naphthalene ring, and an anthracene ring.

Examples of the heterocyclic ring include monocyclic rings such as a pyrrolidine ring, a pyrrole ring, a piperazine ring, a morpholine ring, a thiomorpholine ring, a tetrahydropyridine ring, a lactone ring, and a lactam ring; and fused rings such as a carbazole ring and an indole ring.

The ring formed by R¹ and R², R³ and R⁴, R⁴ and R⁵, R⁵ and R⁶, or a plurality of R⁷ bonded together is preferably a hydrocarbon ring in view of, for example, excellent heat resistance and excellent solvent resistance, and is more preferably an aromatic hydrocarbon ring, in which at least one of the constituent rings is an aromatic ring, and even more preferably an indane ring.

The formulas (IIα) to (IIγ) indicate that two R⁷ in the formula (I) are bonded together to form an indane ring fused to the pyridine ring shown in the formula (I).

Examples of the hydrocarbon group represented by R¹¹ include an alkyl group, an alkenyl group, and an aromatic ring group.

Examples of the alkyl group include various alkyl groups listed above as examples of R¹ and others.

Examples of the alkenyl group include: chain alkenyl groups such as vinyl, ethylene, 2-propenyl, 3-butenyl, 2-butenyl, 4-pentenyl, 3-pentenyl, 2-hexenyl, 3-hexenyl, 5-hexenyl, 2-heptenyl, 3-heptenyl, 4-heptenyl, and 4,8,12-tetradecatrienylallyl; and cyclic alkenyl groups such as cyclopentadienyl.

Examples of the aromatic ring group include groups listed above as examples of R¹ and others.

In the alkyl group having 1 to 20 carbon atoms, the aromatic ring group having 6 to 20 carbon atoms, the heterocyclic group having 2 to 20 carbon atoms, the alkyl group having 1 to 20 carbon atoms with replacement of methylene, the aromatic ring group having 6 to 20 carbon atoms with replacement of methylene, and the heterocyclic group having 2 to 20 carbon atoms with replacement of methylene, which are represented by R¹ to R⁷ and R²¹ to R²⁶, a hydrogen atom may be replaced with a substituent, and examples of the substituent in such a case include a reactive group selected from <Group A> above, and also a halogen atom and a nitro group.

Herein, in a case in which a group having a predetermined number of carbon atoms has a substituent, the predetermined number of carbon atoms also includes that of the substituent, unless specified, for example, with the term "ring-constituting".

For example, a group substituted with a reactive group may be a group represented by a formula (f) below:

* -L_{f}-(R_{f})t Formula (f)

where L_{f} is a group obtained by removing the number t of hydrogen atoms from an alkyl group having 1 to 20 carbon atoms, an aromatic ring group having 6 to 20 carbon atoms, a heterocyclic group having 2 to 20 carbon atoms, a group obtained by removing the number t of hydrogen atoms from an alkyl group having 1 to 20 carbon atoms with replacement of methylene, an aromatic ring group having 6 to 20 carbon atoms with replacement of methylene, or a heterocyclic group having 2 to 20 carbon atoms with replacement of methylene, R_{f} is a reactive group, and t is a number of 1 to 10.

*t* is preferably 1 to 8, more preferably 1 to 5, and even more preferably 1 to 3. L_{f} is preferably a group obtained by removing the number t of hydrogen atoms from an alkyl group, an aromatic ring group, an alkyl group with replacement of methylene, or an aromatic ring group with replacement of methylene, more preferably an aromatic ring group or an aromatic ring group with replacement of methylene, and even more preferably an aromatic ring group. These preferences can impart the compound I highly excellent heat resistance and solvent resistance.

The number of carbon atoms of the group represented by the formula (f) is preferably 10 or less, and may be 5 or less, or 3 or less. Such a preference can impart the compound I highly excellent heat resistance and solvent resistance.

Hereinafter, the group represented by the formula (f) and the reactive group will also be collectively referred to as a "reactive group-containing group".

The reactive group-containing group is particularly preferably the reactive group. Such a preference can impart the compound I highly excellent heat resistance and solvent resistance.

The ring group having 2 to 20 ring-constituting carbon atoms and represented by X in the formula (I) is a group having a ring having 2 to 20 ring-constituting carbon atoms and having, on the ring, a bonding site that is bonded to a pyridine ring in the formula (I). Examples of the ring having 2 to 20 ring-constituting carbon atoms include an aromatic ring having 6 to 20 ring-constituting carbon atoms and a heterocyclic ring having 2 to 20 ring-constituting carbon atoms. The aromatic ring having 6 to 20 ring-constituting carbon atoms may be a hydrocarbon ring constituted by carbon and hydrogen. The ring having 2 to 20 ring-constituting carbon atoms may be a monocyclic ring or a fused ring.

Examples of the aromatic ring having 6 to 20 ring-constituting carbon atoms include: monocyclic rings such as a hydrocarbon-based monocyclic ring constituted by a carbon atom and a hydrogen atom, such as a benzene ring; fused rings such as a hydrocarbon-based fused ring, such as a fluorene ring, a naphthalene ring, a phenanthrene ring, an anthracene ring, an indane ring, a pyrene ring, and a benzopyrene ring; and also ring structures having any of these rings linked together by a linking group. As will be described later, X is preferably a hydrocarbon-based fused ring, and more preferably fluorene.

Examples of the heterocyclic ring having 2 to 20 ring-constituting carbon atoms include: hetero-monocyclic rings such as a furan ring, a pyridine ring, a pyrimidine ring, a thiophene ring, a pyrrole ring, an imidazole ring, a thiazole ring, an oxadiazole ring, a pyridine ring, and a pyrazine ring; and fused rings such as a carbazole ring, an indole ring, a dibenzofuran ring, and a dibenzothiophene ring. Examples of the ring group having 2 to 20 ring-constituting carbon atoms further include ring structures having a plurality of rings linked together by a linking group. The bonding site to the pyridine ring in the formula (I) may be located on any ring.

Examples of the linking group in X include a sulfur atom, a nitrogen atom, a carbonyl group, a carbon atom, and a single bond.

If X has a structure including two or more rings linked together, "having 2 to 20 ring-constituting carbon atoms" means the total number of carbon atoms constituting the two or more rings.

Examples of the chain hydrocarbon group having 1 to 20 carbon atoms represented by X in the formula (I) include a group obtained by removing the number (a-1) of hydrogen atoms from, for example, a chain alkyl group or a chain alkenyl group among the groups listed above as R¹¹. Note that a carbon-carbon unsaturated bond such as *-C=C-* is not located at the terminal of an aliphatic hydrocarbon group unless both ends of the bond are each bonded to a carbon atom.

With respect to X, the "group obtained by replacing or more methylene groups in the chain hydrocarbon group having 2 to 20 carbon atoms with a divalent group selected from <Group B>" herein is also referred to as a "chain hydrocarbon group having 2 to 20 carbon atoms with replacement of methylene".

The number of carbon atoms of the chain hydrocarbon group or the chain hydrocarbon group with replacement of methylene represented by X is within a range of 1 to 20, and is preferably 1 to 15, more preferably 1 to 10, and even more preferably 1 to 5.

The ring having 2 to 20 carbon atoms represented by X may be substituted with a substituent, and examples of the substituent in such a case include a reactive group selected from <Group A> above and the group represented by the formula (f), and also a nitro group.

A hydrogen atom in the chain hydrocarbon group having 1 to 20 carbon atoms may be replaced with a substituent, and examples of the substituent in such a case include a reactive group selected from <Group A> above, and also a nitro group.

Preferred embodiments of the compound I will be further described below. Note that the term "reactive group-containing group" mentioned below can be replaced with the term "reactive group", independently in all occurrences, and further replaced with the term "carbon-carbon unsaturated bond-containing group" or the term "propargyl group or group containing the same (e.g., propargyl group or propargyloxy group)".

The reactive group in the compound I is preferably a carbon-carbon unsaturated bond-containing group, a cyclic ether group, a hydroxyl group, or an amino group, and more preferably a carbon-carbon unsaturated bond-containing group. When the reactive group is a group as mentioned above, a cured product excellent in heat resistance and solvent resistance can be obtained.

The compound I preferably has one or more carbon-carbon unsaturated bond-containing groups in its molecule, and, in such a case, it may or may not have a reactive group different from the carbon-carbon unsaturated bond-containing group. The amino group may be a primary amino group (-NH₂ group), or a secondary amino group substituted with a lower alkyl group having 1 to 3 carbon atoms or an acetyl group, for example, and the amino group is preferably a primary amino group or a secondary amino group substituted with a lower alkyl group.

In the compound I, the number of reactive groups contained in its molecule is 1 or more, and is preferably 2 or more, and more preferably 3 or more. When the number of reactive groups contained in the molecule is within the above-mentioned range, a cured product excellent in heat resistance and solvent resistance can be obtained. In view of ease of synthesis and good storage stability, the number of reactive groups contained in the molecule is preferably 10 or less, and, from the same viewpoint, the number is more preferably 9 or less, and even more preferably 8 or less. For example, in the case of a dipropargylamino group, the number of reactive groups here is 2.

In particular, it is preferable for the compound I that (a) the number of reactive groups contained in the molecule should be 3 or more, or that (b) X should have an optionally substituted fused ring, in view of high heat resistance of a cured product obtained from the compound I, and it is more preferable that both conditions (a) and (b) should be satisfied.

The group represented by R¹ to R⁶ in the formula (I) is preferably a hydrogen atom; a reactive group selected from <Group A> above; or an alkyl group having 1 to 20 carbon atoms, an aromatic ring group having 6 to 20 carbon atoms, an alkyl group having 1 to 20 carbon atoms with replacement of methylene, or an aromatic ring group having 6 to 20 carbon atoms with replacement of methylene, each optionally substituted with a reactive group. The group represented by R¹ to R⁶ is more preferably a hydrogen atom; a reactive group; or an alkyl group having 1 to 20 carbon atoms, an aromatic ring group having 6 to 20 carbon atoms, or an alkyl group having 1 to 20 carbon atoms with replacement of methylene, each optionally substituted with a reactive group. The group represented by R¹ to R⁶ is even more preferably a hydrogen atom; a reactive group; or an alkyl group having 1 to 10 carbon atoms, an aromatic ring group having 6 to 10 carbon atoms, or a alkyl group having 1 to 10 carbon atoms with replacement of methylene, each optionally substituted with a reactive group. The group represented by R¹ to R⁶ is still more preferably a hydrogen atom; a reactive group; or an alkyl group having 1 to 5 carbon atoms or a alkyl group having 1 to 5 carbon atoms with replacement of methylene, each substituted with a reactive group; and still more preferably a hydrogen atom; a reactive group; an alkyl group having 1 or 2 carbon atoms, or a alkyl group having 1 or 2 carbon atoms with replacement of methylene.

When the group represented by R¹ to R⁶ in the general formula (I) is a group as mentioned above, a cured product excellent in heat resistance and solvent resistance can be obtained.

In particular, one or more groups represented by R¹ to R⁶ are preferably reactive group-containing groups. When one or more groups represented by R¹ to R⁶ in the general formula (II) are groups as mentioned above, a cured product excellent in heat resistance and solvent resistance can be obtained.

The total number of reactive groups in R¹ to R⁶ in the formula (I) is preferably 1 to 4, more preferably 1 to 3, and even more preferably 2 or 3. When the number of reactive groups in R¹ to R⁶ in the general formula (I) is within the above-mentioned range, a cured product excellent in heat resistance and solvent resistance can be obtained.

In R¹ to R⁶ in the formula (I), it is preferable that one or more of R¹, R², R⁴, and R⁵ should be reactive group-containing groups. It is more preferable that one or more of R¹, R², and R⁵ should be reactive group-containing groups, and it is even more preferable that either one or both of R¹ and R² should be reactive group-containing groups. In a case in which either one or both of R¹ and R² are reactive group-containing groups, it is preferable that either one or both of R⁴ and R⁵ should be reactive group-containing groups, and it is more preferable that either one of R⁴ and R⁵ should be a reactive group-containing group. Furthermore, in this case, it is preferable that both R¹ and R² should be reactive group-containing groups. When the reactive group-containing group is located as mentioned above in the formula (I), such a compound can give a cured product excellent in heat resistance and solvent resistance, and is also excellent in the film-forming property.

If neither R¹ nor R² is a reactive group-containing group, at least one of R⁴ and R⁵ is preferably a reactive group-containing group in view of heat resistance.

Groups at positions other than the positions preferred for the reactive group-containing groups described above may or may not be reactive group-containing groups.

Among the groups represented by the R¹ to R⁶ in the formula (I), those that are not reactive group-containing groups are each preferably a hydrogen atom, an alkyl group having 1 to 20 carbon atoms, an aromatic ring group having 6 to 20 carbon atoms, an alkyl group having 1 to 20 carbon atoms with replacement of methylene, or an aromatic ring group having 6 to 20 carbon atoms with replacement of methylene; more preferably a hydrogen atom, an alkyl group having 1 to 20 carbon atoms, or a alkyl group having 1 to 20 carbon atoms with replacement of methylene; even more preferably a hydrogen atom, an alkyl group having 1 to 5 carbon atoms, or a alkyl group having 1 to 5 carbon atoms with replacement of methylene; and still more preferably a hydrogen atom, an alkyl group having 1 or 2 carbon atoms, or a alkyl group having 1 or 2 carbon atoms with replacement of methylene. It is also preferable that among groups represented by the R¹ to R⁶ in the formula (I), one or more groups should be hydrogen atoms, in view of ease of synthesis and excellent storage stability of the compound.

In the formula (I), *a* is preferably 1 to 5, more preferably 1 to 3, and even more preferably 1 or 2. When *a* in the formula (I) is a value as mentioned above, the compound is easy to synthesize and has excellent storage stability.

The ring formed by two R⁷ bonded together is preferably a hydrocarbon ring composed only of carbon atoms and hydrogen atoms, more preferably an aromatic hydrocarbon ring, and even more preferably an indane ring fused to the pyridine ring in the formula (I), in view of, for example, its excellent heat resistance. Therefore, the compound I is preferably any of the compounds IIα to IIγ.

In particular, the compound I is more preferably the compound IIα or the compound IIγ, and even more preferably the compound IIα. When the compound of the present invention has a structure as mentioned above, a cured product excellent in heat resistance and solvent resistance can be obtained.

The preferred groups for R²¹, R²², R²³, R²⁴, R²⁵, and R²⁶ are the same as the above-mentioned preferred groups for R¹, R², R³, R⁴, R⁵, and R⁶ respectively.

One or more groups represented by R²¹ to R²⁶ are preferably reactive group-containing groups, in view of obtaining a cured product having excellent heat resistance. To the preferred total number of reactive groups in R²¹ to R²⁶, the preferred total number of reactive groups in R¹ to R⁶ can be applied.

In R²¹ to R²⁶, it is preferable that one or more of R²¹, R²², R²⁴, and R²⁵ should be reactive group-containing groups. It is more preferable that one or more of R²¹, R²², and R²⁵ should be reactive group-containing groups, and it is even more preferable that either one or both of R²¹ and R²² should be reactive group-containing groups. In a case in which either one or both of R²¹ and R²² are reactive group-containing groups, it is preferable that either one or both of R²⁴ and R²⁵ should be reactive group-containing groups, and it is more preferable that either one of R²⁴ and R²⁵ should be a reactive group-containing group. Furthermore, in this case, it is preferable that both of R²¹ and R²² should be reactive group-containing groups. In these cases, the compound can give a cured product excellent in heat resistance and solvent resistance, and is also excellent in the film-forming property.

In R²¹ to R²⁶, if neither R²¹ nor R²² is a reactive group-containing group, at least one of R²⁴ and R²⁵ is preferably a reactive group-containing group in view of heat resistance and solvent resistance. Groups at positions other than the positions preferred for the reactive groups described above may or may not be reactive group-containing groups.

When any group represented by R²¹ to R²⁶ in the formula (I) is not a reactive group-containing group, the group is preferably those preferred for R¹ to R⁶ described above.

X is preferably a ring group having 2 to 20 ring-constituting carbon atoms. Such a preference can impart the compound I highly excellent heat resistance and solvent resistance.

In a case in which X is a ring group having 2 to 20 ring-constituting carbon atoms, the number of the ring-constituting carbon atoms is preferably 3 to 18, more preferably 4 to 15, and even more preferably 6 to 16. Such a preference can impart the compound I highly excellent heat resistance and solvent resistance.

X is more preferably a hydrocarbon-based aromatic ring group or an aromatic heterocyclic group, and even more preferably a hydrocarbon-based aromatic ring group. In that case, the compound gives a cured product excellent in heat resistance and solvent resistance.

The ring in the ring group represented by X in the formula (I) is preferably an optionally substituted fused ring. In view of particularly high heat resistance of a cured product obtained from the compound I, it is preferable that X should have a fused ring optionally substituted, or have an amino group having two reactive groups bonded thereto, and it is more preferable that X should have a fused ring optionally substituted. Examples of the amino group having two reactive groups bonded thereto include a dipropargylamino group. The fused ring is preferably a fused ring constituted by 2 to 6 rings, and more preferably a fused ring constituted by 2 to 4 rings. When the group represented by X in the formula (I) is a fused ring as mentioned above, a cured product having particularly excellent heat resistance can be obtained. For example, fluorene is constituted by 3 rings, naphthalene is constituted by 2 rings, and pyrene is constituted by 4 rings.

Specific examples of X include the formulas (X1) to (X7) below. X is preferably a fused ring represented by any one of the formulas (X1) to (X6) in view of particularly excellent heat resistance.

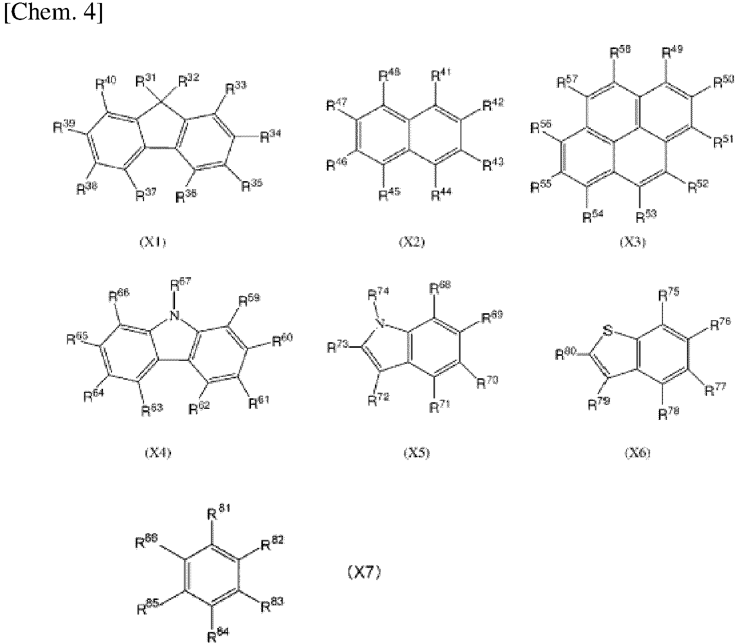

**In** the formulas, R³¹, R³², R³³, R³⁴, R³⁵, R³⁶, R³⁷, R³⁸, R³⁹, R⁴⁰, R⁴¹, R⁴², R⁴³, R⁴⁴, R⁴⁵, R⁴⁶, R⁴⁷, R⁴⁸, R⁴⁹, R⁵⁰, R⁵¹, R⁵², R⁵³, R⁵⁴, R⁵⁵, R⁵⁶, R⁵⁷, R⁵⁸, R⁵⁹, R⁶⁰, R⁶¹, R⁶², R⁶³, R⁶⁴, R⁶⁵, R⁶⁶, R⁶⁷, R⁶⁸, R⁶⁹, R⁷⁰, R⁷¹, R⁷², R⁷³, R⁷⁴, R⁷⁵, R⁷⁶, R⁷⁷, R⁷⁸, R⁷⁹, R⁸⁰, R⁸¹, R⁸², R⁸³, R⁸⁴, R⁸⁵, and R⁸⁶ (hereinafter also referred to as "R³¹ to R⁸⁶") each independently represent a hydrogen atom, a reactive group selected from <Group A> above, a bonding site, a nitro group, an optionally substituted alkyl group having 1 to 20 carbon atoms, an optionally substituted aromatic ring group having 6 to 20 carbon atoms, an optionally substituted heterocyclic group having 2 to 20 carbon atoms, an optionally substituted alkyl group having 1 to 20 carbon atoms with replacement of methylene, an optionally substituted aromatic ring group having 6 to 20 carbon atoms with replacement of methylene, or an optionally substituted heterocyclic group having 2 to 20 carbon atoms with replacement of methylene,
provided that one of R³¹, R³², R³³, R³⁴, R³⁵, R³⁶, R³⁷, R³⁸, R³⁹, and R⁴⁰ is a bonding site, one of R⁴¹, R⁴², R⁴³, R⁴⁴, R⁴⁵, R⁴⁶, R⁴⁷, and R⁴⁸ is a bonding site, one of R⁴⁹, R⁵⁰, R⁵¹, R⁵², R⁵³, R⁵⁴, R⁵⁵, R⁵⁶, R⁵⁷, and R⁵⁸ is a bonding site, one of R⁵⁹, R⁶⁰, R⁶¹, R⁶², R⁶³, R⁶⁴, R⁶⁵, R⁶⁶, and R⁶⁷ is a bonding site, one of R⁶⁸, R⁶⁹, R⁷⁰, R⁷¹, R⁷², R⁷³, and R⁷⁴ is a bonding site, one of R⁷⁵, R⁷⁶, R⁷⁷, R⁷⁸, R⁷⁹, and R⁸⁰ is a bonding site, and one of R⁸¹, R⁸², R⁸³, R⁸⁴, R⁸⁵, and R⁸⁶ is a bonding site.

For the formulas (X1) to (X7), examples of the alkyl group, the aromatic ring group, the heterocyclic group, the alkyl group with replacement of methylene, the aromatic ring group with replacement of methylene, and the heterocyclic group with replacement of methylene include those listed above for R¹ etc., and these groups may be substituted with a reactive group, a halogen atom, or a nitro group, for example.

When any group represented by R³¹ to R⁸⁶ is not a reactive group-containing group or a bonding site, the group is preferably a hydrogen atom, an alkyl group having 1 to 20 carbon atoms, an aromatic ring group having 6 to 20 carbon atoms, an alkyl group having 1 to 20 carbon atoms with replacement of methylene, or an aromatic ring group having 6 to 20 carbon atoms with replacement of methylene; more preferably a hydrogen atom, an alkyl group having 1 to 20 carbon atoms, or an alkyl group having 1 to 20 carbon atoms with replacement of methylene; even more preferably a hydrogen atom, an alkyl group having 1 to 5 carbon atoms, or an alkyl group having 1 to 5 carbon atoms with replacement of methylene; and still more preferably a hydrogen atom or an alkyl group having 1 or 2 carbon atoms. In the respective rings of the formulas (X1) to (X7), the number of substituents other than hydrogen atom, the bonding site, or the reactive group-containing group is preferably 0 to 2, and more preferably 0 or 1, and may be 0. Such a compound I can form a compound highly excellent in heat resistance and solvent resistance, and is also easy to synthesize and excellent in storage stability.

Preferred modes of each of the formulas (X1) to (X7) will be described below.

A compound in which R³⁴ or R³⁹ in the formula (X1) is a bonding site is preferable, in view of ease of synthesis and excellent storage stability and also obtaining a cured product excellent in heat resistance.

The number of reactive groups in the formula (X1) is preferably 1 to 8, more preferably 1 to 6, and even more preferably 2 to 4. A compound having such a number of reactive groups is easy to synthesize, has excellent storage stability, and can give a cured product excellent in heat resistance and solvent resistance.

In the formula (X1), it is preferable that one or more selected from R³¹, R³², and R³⁷ to R⁴⁰ should be reactive group-containing groups. It is more preferable that either one or both of R³¹ and R³² should be reactive group-containing groups, and it is even more preferable that both of R³¹ and R³² should be reactive group-containing groups.

A compound in which the reactive group-containing group in the formula (X1) is located as mentioned above has excellent solubility in solvents and can also give a cured product excellent in heat resistance.

A compound in which R⁴⁵ in the formula (X2) is a bonding site is preferable in view of ease of synthesis and excellent storage stability and also obtaining a cured product excellent in heat resistance.

A compound having one to six reactive groups in the formula (X2) is preferable, and a compound having one to four reactive groups, particularly one or two reactive groups, in the formula (X2) is more preferable. A compound having the above-mentioned number of reactive groups is easy to synthesize and excellent in storage stability, and can also give a cured product excellent in heat resistance.

In the formula (X2), it is preferable that R⁴⁶, R⁴⁷, or R⁴⁸ should be a reactive group-containing group, and it is more preferable that R⁴⁶ should be a reactive group-containing group, in view of excellent solubility in solvents and also obtaining a cured product excellent in heat resistance.

A compound in which R⁵⁴ in the formula (X3) is a bonding site is preferable in view of ease of synthesis and excellent storage stability and also obtaining a cured product excellent in heat resistance. R⁴⁹, R⁵⁰, R⁵¹, R⁵², R⁵³, R⁵⁴, R⁵⁵, R⁵⁶, R⁵⁷, and R⁵⁸ may or may not be independently a reactive group-containing group.

A compound in which R⁶¹ or R⁶⁴ in the formula (X4) is a bonding site is preferable in view of ease of synthesis and excellent storage stability and also obtaining a cured product excellent in heat resistance.

A compound in which R⁷² in the formula (X5) is a bonding site is preferable in view of ease of synthesis and excellent storage stability and also obtaining a cured product excellent in heat resistance.

A compound in which R⁷⁴ in the formula (X5) is a reactive group-containing group is preferable in view of excellent solubility in solvents and also obtaining a cured product excellent in heat resistance.

A compound in which R⁸⁰ in the formula (X6) is a bonding site is preferable in view of ease of synthesis and excellent storage stability and also obtaining a cured product excellent in heat resistance.

In the formula (X7), the number of reactive groups is preferably 1 to 3, and more preferably 1 or 2. In that case, a cured product excellent in heat resistance and solvent resistance can be obtained.

In X, groups at positions other than the positions preferred for the reactive group-containing groups or bonding sites described above may or may not be reactive group-containing groups.

The ring group represented by X in the formula (I) is preferably at least one selected from a fluorene ring, a benzene ring, a naphthalene ring, a carbazole ring, and a pyrene ring, and more preferably at least one selected from a fluorene ring, a benzene ring, a naphthalene ring, and a pyrene ring, in view of heat resistance. The ring group represented by X in the formula (I) is even more preferably at least one selected from a fluorene ring, a naphthalene ring, and a pyrene ring, and still more preferably a fluorene ring.

The bonding site of the group represented by X in the formula (I) is preferably at the para position relative to the nitrogen atom of the pyridine ring, in view of ease of synthesis and excellent storage stability.

Examples of the compound I include, but are not limited to, compounds (101) to (179) below as those having a propargyl group, a propargyloxy group, an ethynyl group, or a vinyl group as the reactive group.

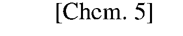

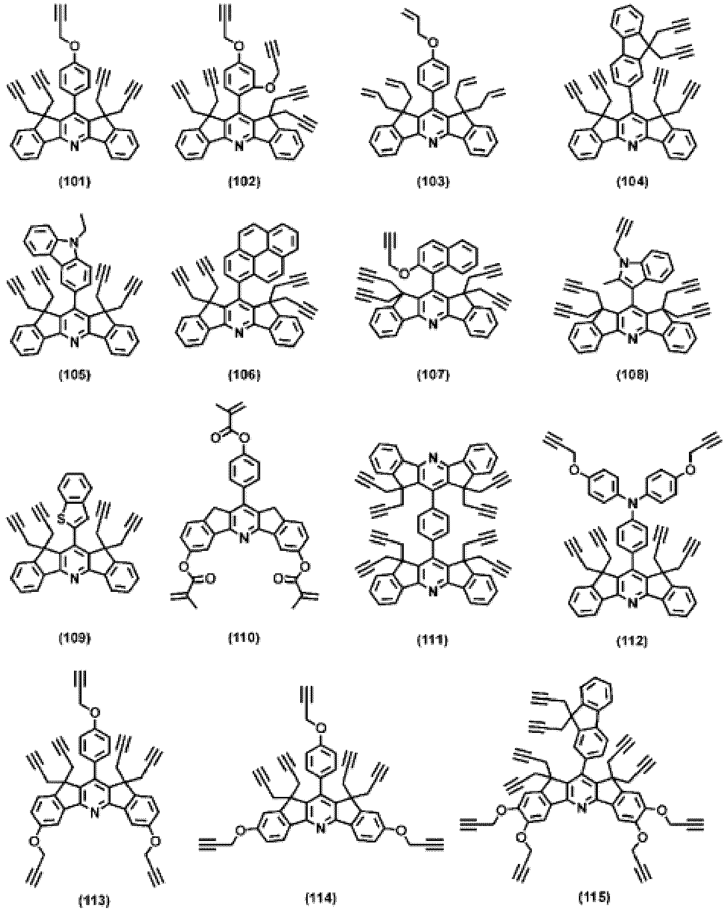

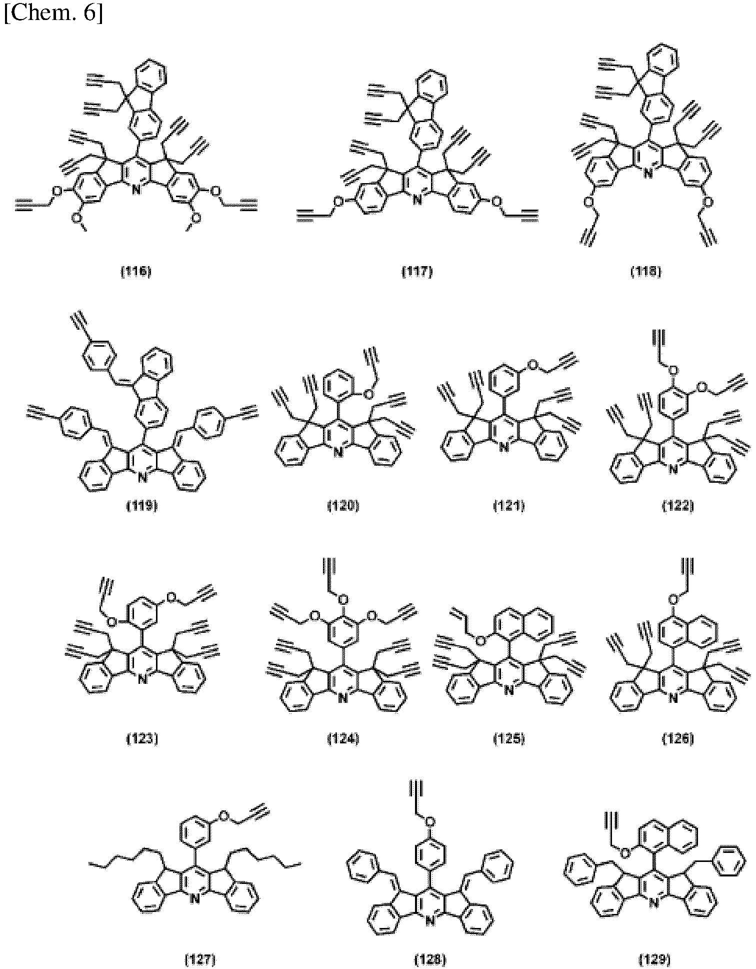

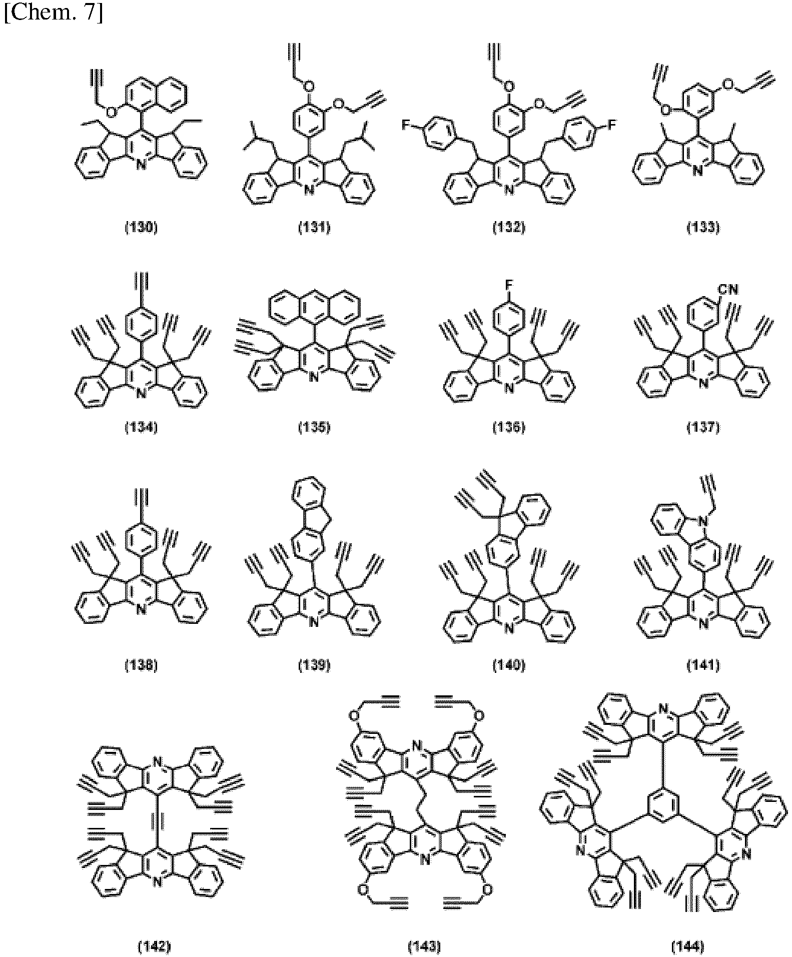

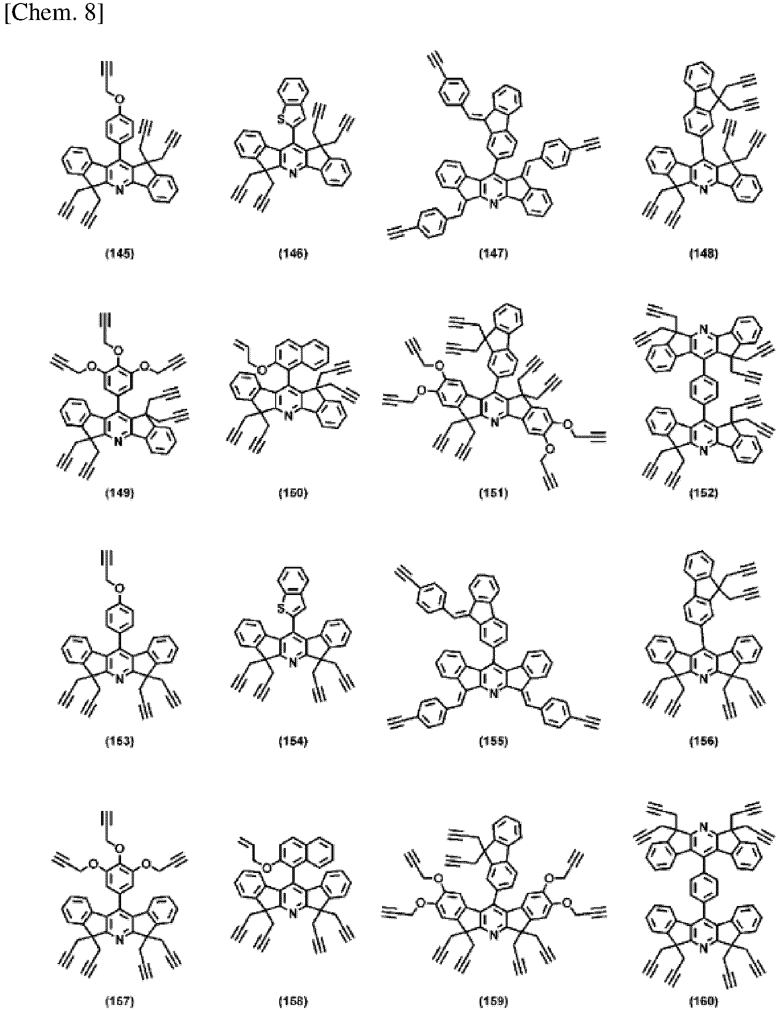

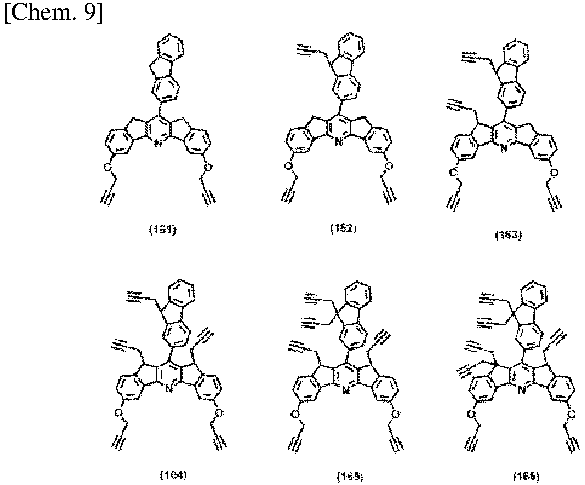

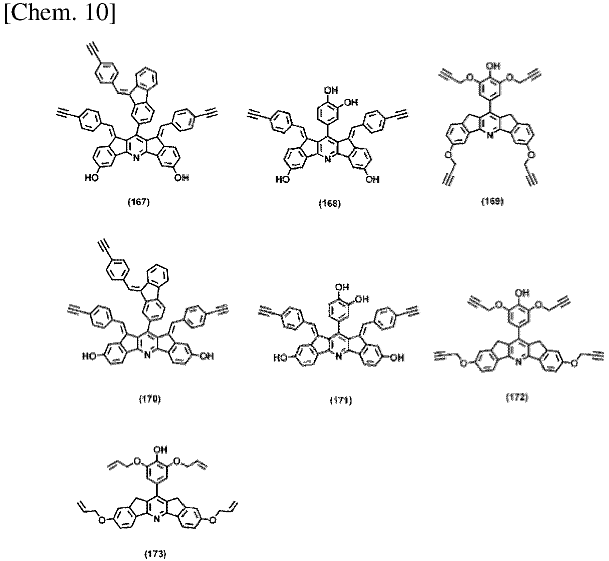

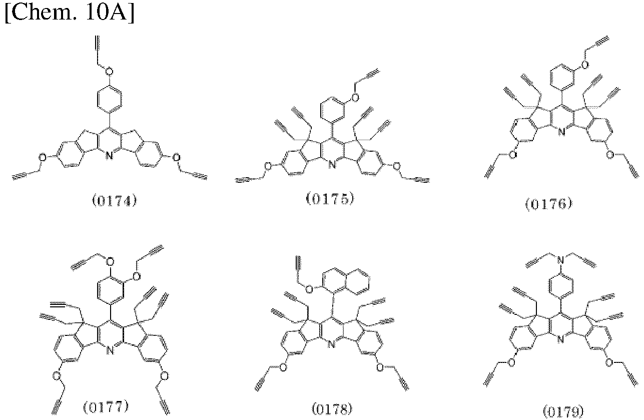

Next, the compound represented by the general formula (III) above (hereinafter also referred to as a "compound III") will be described. The compound III can be used as a precursor of the compound I.

In the compound III, X', R¹', R²', R³', R⁴', R⁵', R⁶', R⁷', R¹¹', and *a*' are the same as defined for X, R¹, R², R³, R⁴, R⁵, R⁶, R⁷, R¹¹, and *a*, respectively, in the compound I, and the foregoing description for X, R¹, R², R³, R⁴, R⁵, R⁶, R⁷, R¹¹, and *a* (hereinafter also referred to as "X to *a*") in the compound I can be entirely applied to X', R¹', R²', R³', R⁴', R⁵', R⁶', R⁷', R¹¹', *a*' (hereinafter also referred to as "X' to *a*'") respectively, except for the preferred type or number of the reactive groups. For example, as with X, X' is preferably an optionally substituted aromatic ring group having 6 to 20 carbon atoms or an optionally substituted heterocyclic group having 2 to 20 carbon atoms. X' is also preferably a fused ring group optionally substituted or preferably has a primary amino group, and more preferably a fused ring group optionally substituted. The compound III will now be described in detail below. If there is any discrepancies between the foregoing description for X to *a* and the following description for X' to *a*' of the compound III, preference is given to the following description for X' to *a*'. The compound III is different from the compound I in that the compound III necessarily has at least one phenolic hydroxyl group.

The phenolic hydroxyl group refers to a hydroxyl group bonded directly to an aromatic ring, and is preferably a hydroxyl group bonded directly to a benzene ring that constitutes a fused ring or a benzene ring that is a monocyclic ring.

Preferred examples of the compound III include the general formulas (IIIα) to (IIIγ) below.

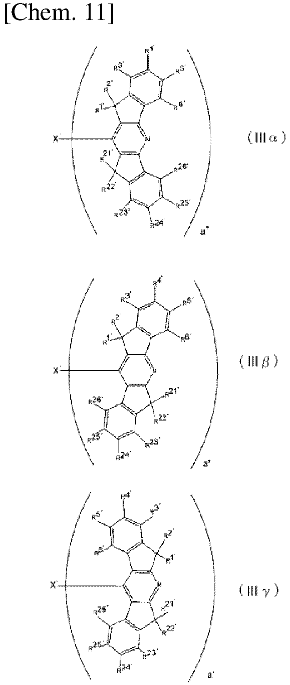

In the formulas, X', *a*', R¹', R²', R³', R⁴', R⁵', and R⁶' are as defined for the general formula (III),

R²¹', R²²', R²³', R²⁴', R²⁵', and R²⁶' (hereinafter also referred to as "R²¹' to R²⁶' ") each independently represent a hydrogen atom, a reactive group selected from <Group A> above, a nitro group, an optionally substituted alkyl group having 1 to 20 carbon atoms, an optionally substituted aromatic ring group having 6 to 20 carbon atoms, an optionally substituted heterocyclic group having 2 to 20 carbon atoms, a group obtained by replacing one or more methylene groups in the alkyl group having 1 to 20 carbon atoms with a divalent group selected from <Group B> above, a group obtained by replacing one or more methylene groups in the aromatic ring group having 6 to 20 carbon atoms with a divalent group selected from <Group B> above, or a group obtained by replacing one or more methylene groups in the heterocyclic group having 2 to 20 carbon atoms with a divalent group selected from <Group B> above,
R²¹' and R²²' may be bonded together to form a ring, R²³' and R²⁴, may be bonded together to form a ring, R²⁴' and R²⁵' may be bonded together to form a ring, and R²⁵' and R²⁶' may be bonded together to form a ring,
provided that one or more phenolic hydroxyl groups are contained in the molecule.

The description and preferred examples for groups R²¹' to R²⁶' are the same as those for R²¹ to R²⁶, respectively.

Preferred embodiments of the compound III will be described below. In the description below, the term "phenolic hydroxyl group-containing group" is a collective term for a phenolic hydroxyl group and a group represented by the formula (f') below. Note that the term "phenolic hydroxyl group-containing group" mentioned below can be replaced, in all occurrences independently, with the term "phenolic hydroxyl group", which is the subordinate concept.

* -L_{f}'-(OHₚ)t' Formula (f')

In the formula, L_{f}' is an aromatic ring-containing group having 6 to 20 carbon atoms, OHₚ is a phenolic hydroxyl group, and t' is a number of 1 to 10.

The aromatic ring-containing group having 6 to 20 carbon atoms is a group obtained by removing the number t' of hydrogen atoms from an arylalkyl group having 7 to 20 carbon atoms, an aromatic hydrocarbon ring-containing heterocyclic group having 8 to 20 carbon atoms, or a group obtained by removing the number t' of hydrogen atoms from an aryl group having 6 to 20 carbon atoms, and preferably has 6 to 10 carbon atoms, and is more preferably a phenylene group having 6 to 10 carbon atoms and optionally substituted with an alkyl group or an alkoxy group. Examples of the aromatic hydrocarbon ring-containing heterocyclic group having 8 to 20 carbon atoms include a ring composed of a benzene ring and a heterocyclic ring fused together, such as indole or carbazole.

t' is preferably 1 to 8, more preferably 1 to 5, even more preferably 1 to 3, and still more preferably 1 or 2, and t' may be 1.

The reactive group(s) of the compound III include(s) a phenolic hydroxyl group, and may or may not include a reactive group different from a phenolic hydroxyl group. For the case where the compound III has a reactive group different from a phenolic hydroxyl group, examples of the reactive group include an amino group.

A compound having the group(s) as mentioned above as the reactive group serves as a precursor for successively producing the compound I.

In the compound III, the number of phenolic hydroxyl groups contained in its molecule is preferably 1 or more, and more preferably 2 or more. When the number of phenolic hydroxyl groups contained in the molecule is within the above-mentioned range, such a compound III is advantageously used as a precursor and also advantageously gives a cured product excellent in heat resistance and solvent resistance. In view of ease of synthesis and good storage stability, the number of phenolic hydroxyl groups contained in the molecule is preferably 10 or less, and, from the same viewpoint, the number is more preferably 8 or less, and even more preferably 6 or less.

The group represented by R¹' to R⁶' in the formula (III) is preferably a hydrogen atom; a phenolic hydroxyl group; or an alkyl group having 1 to 20 carbon atoms, an aromatic ring group having 6 to 20 carbon atoms, an alkyl group having 1 to 20 carbon atoms with replacement of methylene, or an aromatic ring group having 6 to 20 carbon atoms with replacement of methylene, each optionally having a phenolic hydroxyl group. The group represented by R¹' to R⁶' is more preferably a hydrogen atom; a phenolic hydroxyl group; or an alkyl group having 1 to 10 carbon atoms, an aromatic ring group having 6 to 10 carbon atoms, or a alkyl group having 1 to 10 carbon atoms with replacement of methylene, each optionally having a phenolic hydroxyl group. The group represented by R¹' to R⁶' is even more preferably a hydrogen atom, a phenolic hydroxyl group, an alkyl group having 1 to 5 carbon atoms, or a alkyl group having 1 to 5 carbon atoms with replacement of methylene; and still more preferably a hydrogen atom, a phenolic hydroxyl group, an alkyl group having 1 or 2 carbon atoms, or an alkoxy group having 1 or 2 carbon atoms.

It is preferable that at least one of R³', R⁴', R⁵', R⁶', R²³', R²⁴', R²⁵', and R²⁶' should be a phenolic hydroxyl group-containing group, and it is more preferable that at least two of them should be phenolic hydroxyl group-containing groups. It is even more preferable that at least one of R³', R⁴', R⁵', and R⁶' should be a phenolic hydroxyl group-containing group and at least one of R²³', R²⁴', R²⁵', and R²⁶' should be a phenolic hydroxyl group-containing group. In particular, the phenolic hydroxyl group-containing groups mentioned here are preferably phenolic hydroxyl groups.

It is preferable that at least two of R⁴', R⁵', R²⁴', and R²⁵' should be phenolic hydroxyl group-containing groups, and, in particular, these phenolic hydroxyl group-containing groups are preferably phenolic hydroxyl groups.

In particular, one or more groups represented by R¹' to R⁶' are preferably phenolic hydroxyl group-containing groups. When the group represented by R¹' to R⁶' are a group as mentioned above, such a compound III is advantageously used as a precursor, and also advantageously gives a cured product excellent in heat resistance and solvent resistance.

The total number of phenolic hydroxyl groups in R¹' to R⁶' in the formula (III) is preferably 3 or less, and more preferably 2 or less, and may be 1. When the number of phenolic hydroxyl groups in R¹' to R⁶' in the formula (III) is within the above-mentioned range, such a compound III is advantageously used as a precursor, and also advantageously gives a cured product excellent in heat resistance and solvent resistance.

In cases where any one of R¹' to R⁶' in the formula (III) has a phenolic hydroxyl group, it is preferable that either one or both of R⁴' and R⁵' should be phenolic hydroxyl group-containing groups, and it is more preferable that either one of R⁴' and R⁵' should be a phenolic hydroxyl group-containing group. The phenolic hydroxyl group-containing groups mentioned here are preferably phenolic hydroxyl groups. When the phenolic hydroxyl group is located at the position as mentioned above in the formula (III), such a compound III advantageously gives a cured product excellent in heat resistance and solvent resistance.

For groups that are not phenolic hydroxyl group-containing groups among the groups represented by R¹' to R⁶' in the formula (III), preferred examples thereof include those listed above as preferred examples of groups that are not phenolic hydroxyl group-containing groups among the groups represented by R¹ to R⁶. The group that is not a phenolic hydroxyl group-containing group is more preferably a hydrogen atom, an alkyl group having 1 or 2 carbon atoms, or an alkoxy group having 1 or 2 carbon atoms.

The preferred groups for R²¹', R²²', R²³', R²⁴', R²⁵', and R²⁶' are the same as the above-mentioned preferred groups for R¹', R²', R³', R⁴', R⁵', and R⁶, respectively, and the foregoing description applies thereto.

One or more groups represented by R²¹' to R²⁶' are preferably phenolic hydroxyl group-containing groups. In that case, such a compound III is advantageously used as a precursor and also advantageously gives a cured product excellent in heat resistance and solvent resistance. The preferred total number of phenolic hydroxyl groups in R²¹' to R²⁶' is the same as described for the preferred total number of phenolic hydroxyl groups in R¹' to R⁶'.

In cases where any of R²¹' to R²⁶' in the formula (III) have a phenolic hydroxyl group, it is preferable that either one or both of R²⁴' and R²⁵' should be phenolic hydroxyl group-containing groups, and it is more preferable that either one of R²⁴' and R²⁵' should be a phenolic hydroxyl group-containing group. In particular, the phenolic hydroxyl group-containing groups mentioned here are preferably phenolic hydroxyl groups. A compound having the phenolic hydroxyl group located at the position as mentioned above in the formula (III) is easy to synthesize and excellent in storage stability.

For groups that are not phenolic hydroxyl group-containing groups among groups represented by R²¹' to R²⁶' in the formula (I), preferred examples thereof may be the same as those described for R¹' to R⁶'.

X' may or may not have a phenolic hydroxyl group. The number of phenolic hydroxyl groups in X' is preferably 0 to 6, more preferably 0 to 4, and even more preferably 0 to 2. When the number of phenolic hydroxyl groups is as mentioned above, such a compound III is advantageously used as a precursor and also advantageously gives a cured product excellent in heat resistance and solvent resistance.

X' may contain an amino group instead of a phenolic hydroxyl group. If X' contains an amino group, the total number of active hydrogens in the amino group and phenolic hydroxyl groups is preferably the same as described above for the number of phenolic hydroxyl groups in X' (similarly, the number of phenolic hydroxyl groups is X'1 to X'7, which will be mentioned below, can be replaced with the total number of active hydrogens in the amino group and phenolic hydroxyl groups).

Specific examples of X' above include groups represented by the formulas (X' 1) to (X'7) below. When the group represented by X' in the formula (III) is a ring represented by any one of the formulas (X' 1) to (X'6), such a compound can be favorably used as a precursor and also give a cured product highly excellent in heat resistance.

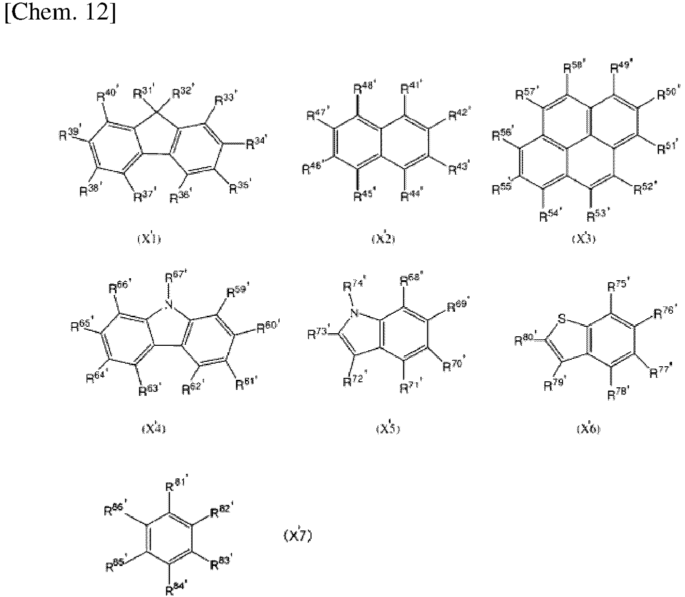

In the formulas, R³¹', R³²', , R³³', R³⁵', R³⁶', R³⁷', R³⁸', R³⁹', R⁴⁰', R⁴¹', R⁴²', R⁴³', R⁴⁴', R⁴⁵, R⁴⁶', R⁴⁷, , R⁴⁸', R⁴⁹', R⁵⁰, R⁵¹, R⁵²', R^{53'}, R⁵⁴, R⁵⁵', R⁵⁶', R⁵⁷, R⁵⁸, R⁵⁹', R⁶⁰', R⁶¹', R⁶²', R⁶³', R⁶⁴', R⁶⁵', R⁶⁶', R⁶⁷', R⁶⁸', R⁶⁹', R⁷⁰', R⁷¹', R⁷²', R⁷³', R⁷⁴', R⁷⁵', R⁷⁶', R⁷⁷', R⁷⁸', R⁷⁹', R⁸⁰', R⁸¹', R⁸²', R⁸³', R⁸⁴', R⁸⁵', and R⁸⁶' (hereinafter also referred to as "R³¹' to R⁸⁶' ") each independently represent a hydrogen atom, a phenolic hydroxyl group, an amino group, a bonding site, a nitro group, an optionally substituted alkyl group having 1 to 20 carbon atoms, an optionally substituted aromatic ring group having 6 to 20 carbon atoms, an optionally substituted heterocyclic group having 2 to 20 carbon atoms, an optionally substituted alkyl group having 1 to 20 carbon atoms with replacement of methylene, an optionally substituted aromatic ring group having 6 to 20 carbon atoms with replacement of methylene, or an optionally substituted heterocyclic group having 2 to 20 carbon atoms with replacement of methylene,
provided that one of R³¹', R³²', R³³', R³⁵', R³⁶', R³⁷', R³⁸', R³⁹', and R⁴⁰' is a bonding site, one of R⁴¹', R⁴²', R⁴³', R⁴⁴', R⁴⁵', R⁴⁶', R⁴⁷', and R⁴⁸' is a bonding site, one of R⁴⁹', R⁵⁰, R⁵¹', R⁵², , R⁵³', R⁵⁴, R⁵⁵', , R⁵⁶, R⁵⁷, and R⁵⁸, is a bonding site, one of R⁵⁹', R⁶⁰', R⁶¹', R⁶²', R⁶³', R⁶⁴', R⁶⁵', R⁶⁶', and R⁶⁷' is a bonding site, one of R⁶⁸', R⁶⁹', R⁷⁰', R⁷¹', R⁷²', R⁷³', and R⁷⁴' is a bonding site, one of R⁷⁵', R⁷⁶', R⁷⁷', R⁷⁸', R⁷⁹', and R⁸⁰' is a bonding site, and one of R⁸¹', R⁸²', R⁸³', R⁸⁴', R⁸⁵', and R⁸⁶', is a bonding site.

For the formulas (X' 1) to (X'7), examples of the alkyl group, the aromatic ring group, the heterocyclic group, the alkyl group with replacement of methylene, the aromatic ring group with replacement of methylene, and the heterocyclic group with replacement of methylene include those listed above for R¹ etc., and these groups may be substituted with a phenolic hydroxyl group, a halogen atom, a nitro group, or an amino group, for example.

X' may or may not have a phenolic hydroxyl group. In a case where X' has a phenolic hydroxyl group, one or more of the groups corresponding to R³¹' to R⁸⁶' in X'1 to X'7, respectively, are preferably phenolic hydroxyl group-containing groups, and more preferably phenolic hydroxyl groups. For example, one or more of the groups represented by R⁸¹' to R³', which are groups corresponding to X'7, are preferably phenolic hydroxyl group-containing groups, and more preferably phenolic hydroxyl groups.

R³¹' to R⁸⁶' correspond to R³¹ to R⁸⁶, respectively.

Preferred groups when any group represented by R³¹ to R⁸⁶ is not a reactive group-containing groups or bonding sites have been described hereinabove, and that description applies as appropriate to preferred groups when any group represented by R³¹' to R⁸⁶' is not a phenolic hydroxyl groups or bonding sites. In the respective rings of the formulas (X' 1) to (X'7), the preferred number of substituents other than hydrogen atoms, the bonding site, the phenolic hydroxyl group-containing group, or the amino group is the same as described for the preferred number of substituents other than hydrogen atoms, the bonding site, or the reactive group-containing group in the respective rings of the formulas (X1) to (X7).

For preferred positions of bonding sites, the description for X1 to X7 applies as appropriate to X'1 to X'7.

In particular, in each of the formulas (X'2) and (X'7), the number of reactive groups (for example, phenolic hydroxyl groups) is preferably 1 to 3, and more preferably 1 or 2. Such a compound can give a cured product excellent in heat resistance and solvent resistance.

In X', groups at positions other than the positions preferred for the phenolic hydroxyl group-containing groups or the bonding site described above may or may not be reactive group-containing groups.

The compound I produced from the compound III as a precursor is preferably those having a propargyloxy group, an allyl ether group, or a glycidyl group as the reactive group.

Examples of the compound III having a phenolic hydroxyl group include compounds (1) to (65) below, in addition to the compounds (168), (169), and (171) to (173) above. Such a compound III can also be used as an intermediate for producing the compound I having a reactive group such as a propargyloxy group.

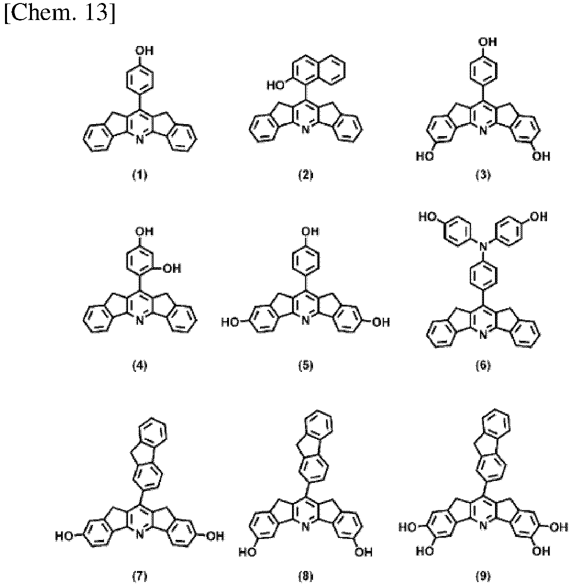

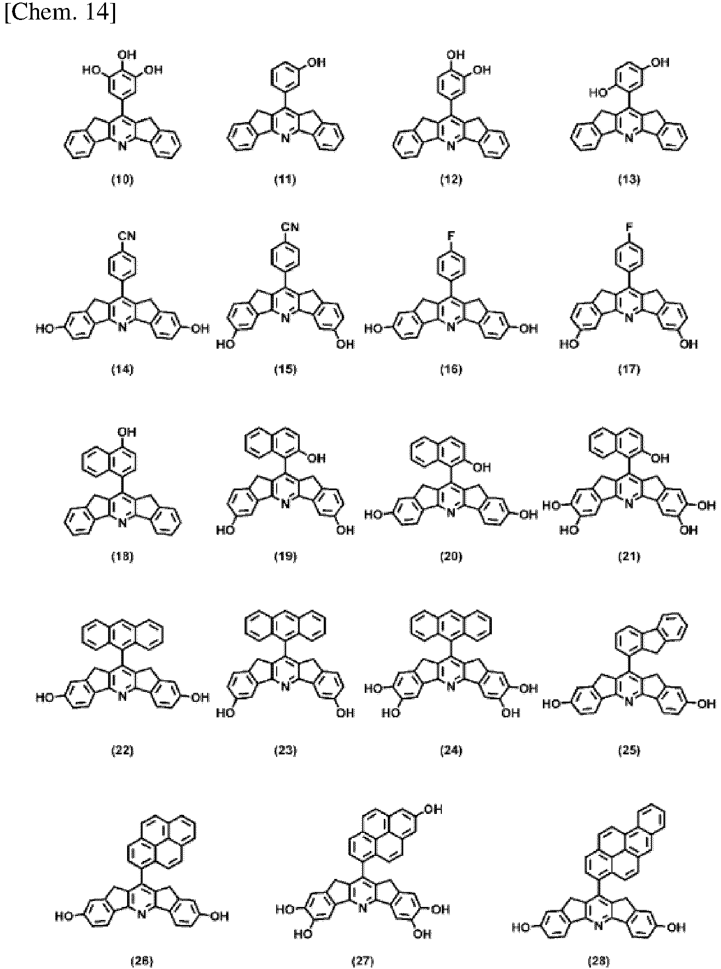

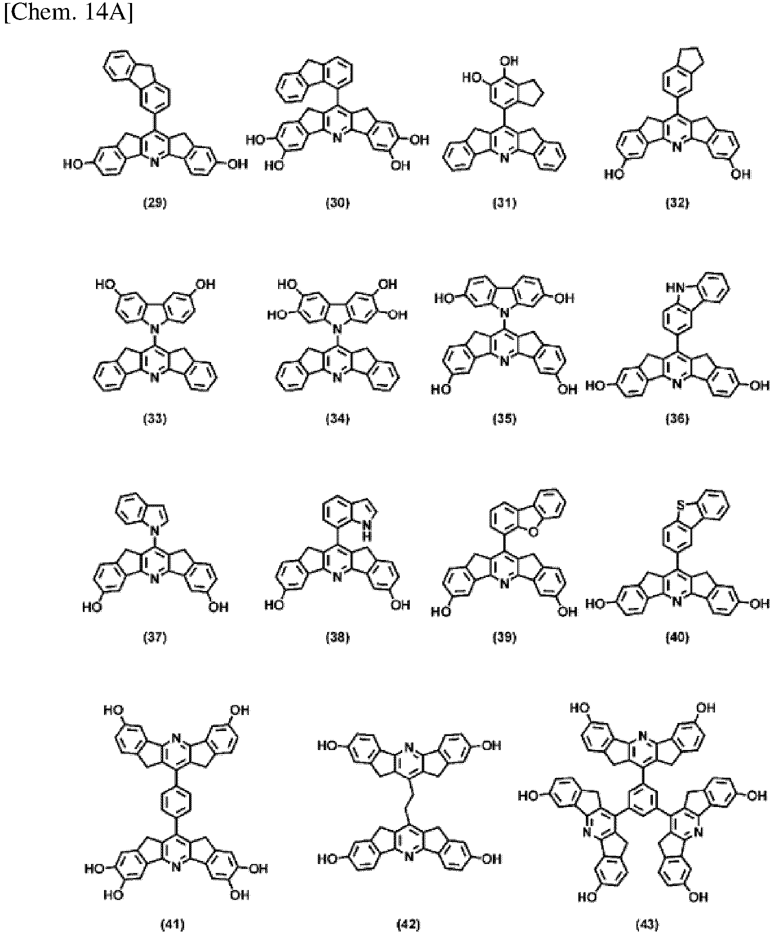

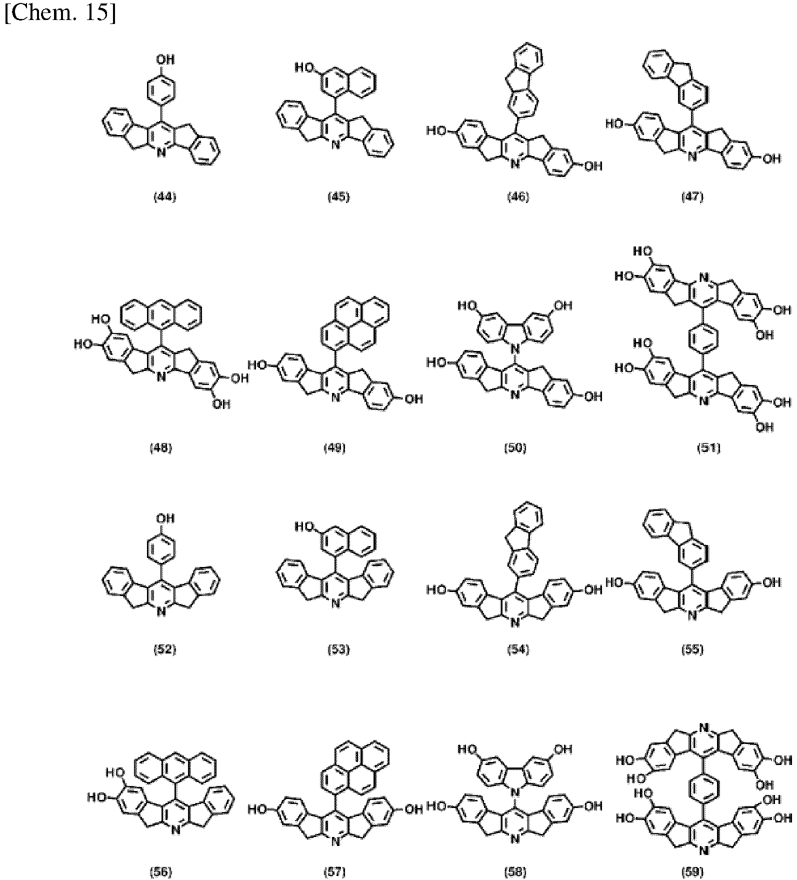

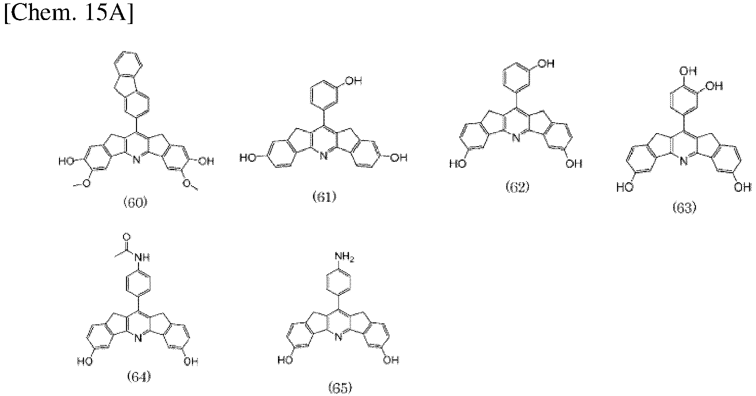

The compound I and the compound III described above can be produced according to known methods. Specifically, an indanone compound and an aldehyde compound can be fused with ammonium acetate to produce a compound having a diindenopyridine skeleton structure, and furthermore, a reactive group can be introduced to that skeletal structure to produce the above-described compound I. For example, in a case where the aldehyde compound or the indanone compound used has a hydroxyl group, the compound III (the compound I having a hydroxyl group as a reactive group) can be produced, and the hydroxyl group in the compound III can be further replaced with a reactive group different from a hydroxyl group by using a halogen compound and a carbonate salt such as K₂CO₃ (e.g., Scheme 1 below).

A reactive group can be introduced to a predetermined position in the indenopyridine skeleton structure in the formula (I), using a halogen compound and an alkaline agent such as NaOH (e.g., Scheme 2 below).

Schemes 1 and 2 below each show an exemplary case where the compound I corresponds to the compound IIα wherein a=1, and the compounds IIβ and IIγ can be produced according to methods for other compounds (see European Journal of Organic Chemistry (2005), (10), p. 2045-2055, or RSC Advances (2015), 5(63), p. 51183-51187, for example). A compound wherein *a* is 2 or more can be produced by using a polyfunctional aldehyde compound.

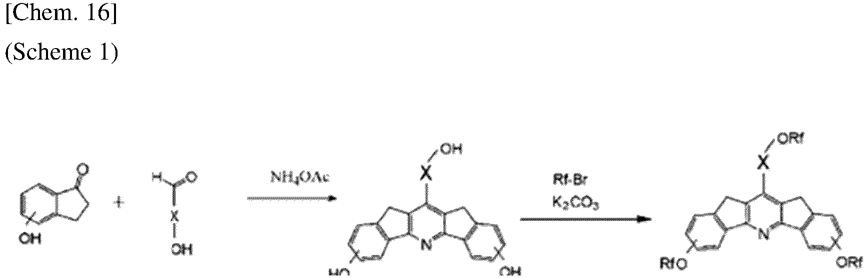

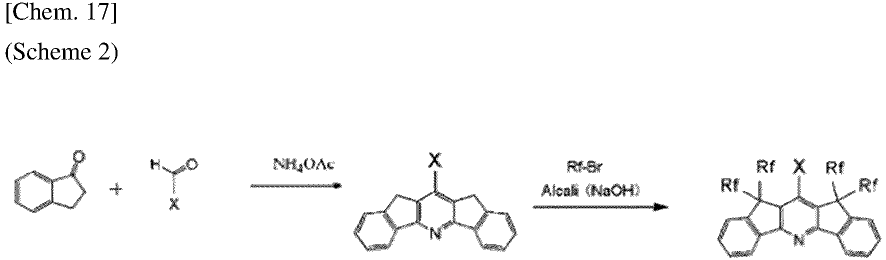

### B. Composition

The composition of the present invention is characterized in that it contains the above-described compound I. The composition, which contains the compound I, can give a cured product having excellent heat resistance.

The composition preferably contains the compound I in an amount of 20 to 100 mass% based on its solids content, in view of obtaining a cured product having excellent heat resistance. The content of the compound I is more preferably 40 to 100% by mass, and even more preferably 50 to 100% by mass, based on the solids content, and may be 60% by mass or more. The term "solids content" here refers to the components of the composition excluding a solvent, which will be described later.

The composition of the present invention may contain a cross-linking agent. The cross-linking agent means a compound that reacts with the compound I to link a plurality of molecules together through chemical bonding and is thus incorporated into a polymer from the polymerization reaction to thereby modify the physical and chemical properties of the polymer. When the composition contains a cross-linking agent, a cured product having highly excellent heat resistance can be obtained. Examples of the cross-linking agent include a phenolic compound, an epoxy compound, a cyanate compound, an amine compound, a benzoxazine compound, a melamine compound, a guanamine compound, a glycoluril compound, a urea compound, an isocyanate compound, and an azide compound.

Examples of the phenolic compound include: alkylphenols such as phenol, cresols, and xylenols; bisphenols such as bisphenol A, bisphenol F, bis(3-methyl-4-hydroxyphenyl)propane, and bis(4-hydroxyphenyl)-1-phenylethane; trisphenols such as α,α,α'-tris(4-hydroxyphenyl)-1-ethyl-4-isopropylbenzene; phenolic resins such as a phenolic novolac resin and a phenolaralkyl resin; and resinous phenolic derivatives such as linear trisphenols, methane-type trisphenols, linear tetrakisphenols, and radial hexanuclear compounds, represented by the general formulas below, respectively.

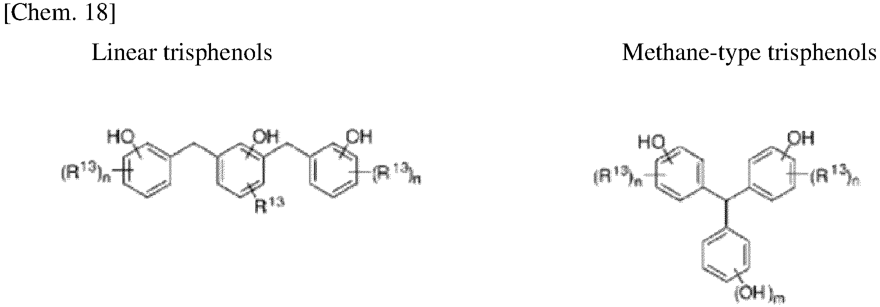

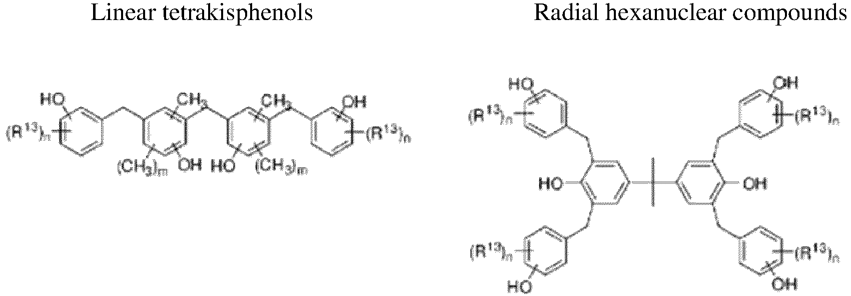

In the formulas, R¹³ represents an alkyl group having 1 to 4 carbon atoms, n represents an integer of 0 to 2, and m represents an integer of 0 or 1.

Examples of the epoxy compound include glycidyl ethers of the above-mentioned phenolic compounds, tris(2,3-epoxypropyl)isocyanurate, trimethylolmethane triglycidyl ethers, and trimethylolpropane triglycidyl ethers.

Examples of the cyanate resin include a compound obtained by replacing a hydroxy group in the above-mentioned phenolic compound with a cyanate group.

Examples of the amine compound include: aromatic amines such as m-phenylenediamine, p-phenylenediamine, 4,4'-diaminodiphenylmethane, 4,4'-diaminodiphenylpropane, 4,4'-diaminodiphenyl ether, and 1,3-bis(4-aminophenoxy)benzene; alicyclic amines such as diaminocyclohexane, diaminodicyclohexylmethane, diaminodicyclohexylpropane, diaminobicyclo[2.2.1]heptane, and isophorone diamine; and aliphatic amines such as ethylenediamine, hexamethylenediamine, octamethylenediamine, decamethylenediamine, diethylenetriamine, and triethylenetetramine.

Examples of the benzoxazine compound include P-d type benzoxazine obtained from a diamine compound and a monofunctional phenolic compound, and F-a type benzoxazine obtained from an amine compound and a bifunctional phenolic compound.

Examples of the melamine compound include: hexamethylolmelamine; hexamethoxymethylmelamine; compounds obtained by methoxymethylating one to six methylol groups of hexamethylolmelamine, or mixtures thereof; and hexamethoxyethylmelamine; hexaacyloxymethylmelamine; compounds obtained by acyloxymethylating one to six methylol groups of hexamethylolmelamine, or mixtures thereof.

Examples of the guanamine compound include: tetramethylolguanamine; tetramethoxymethylguanamine; compounds obtained by methoxymethylating one to four methylol groups of tetramethylolguanamine, or mixtures thereof; tetramethoxyethylguanamine, tetraacyloxyguanamine; and compounds obtained by acyloxymethylating one to four methylol groups of tetramethylolguanamine, or mixtures thereof.

Examples of the glycoluril compound include: tetramethylolglycoluril; tetramethoxyglycoluril; tetramethoxymethylglycoluril; compounds obtained by methoxymethylating one to four methylol groups of tetramethylolglycoluril, or mixtures thereof; and compounds obtained by acyloxymethylating one to four methylol groups of tetramethylolglycoluril, or mixtures thereof.

Examples of the urea compound include: tetramethylolurea; tetramethoxymethylurea; compounds obtained by methoxymethylating one to four methylol groups of tetramethylolurea, or mixtures thereof; and tetramethoxyethylurea.

Examples of the isocyanate compound include: tolylene diisocyanate, diphenylmethane diisocyanate, hexamethylene diisocyanate, and cyclohexane diisocyanate. Examples of the azide compound include: 1,1'-biphenyl-4,4'-bisazide, 4,4'-methylidenebisazide, and 4,4'-oxybisazide.

As the cross-linking agent, the phenolic compound or the glycoluril compound is preferably used, in view of obtaining a cured product having excellent heat resistance.

The content of the cross-linking agent is preferably 0.1 to 20 parts by mass, and more preferably 1 to 10 parts by mass, per 100 parts by mass of the compound I.

The composition of the present invention may contain a solvent. Usually, a solvent that can dissolve or disperse the above-described components as necessary can be used as the solvent. Examples include: ketones such as methyl ethyl ketone, methyl amyl ketone, diethyl ketone, acetone, methyl isopropyl ketone, methyl isobutyl ketone, cyclohexanone, and 2-heptanone; ether solvents such as ethyl ether, dioxane, tetrahydrofuran, 1,2-dimethoxyethane, 1,2-diethoxyethane, and dipropylene glycol dimethyl ether; ester solvents such as methyl acetate, ethyl acetate, n-propyl acetate, isopropyl acetate, n-butyl acetate, cyclohexyl acetate, ethyl lactate, dimethyl succinate, and Texanol; Cellosolve solvents such as ethylene glycol monomethyl ether and ethylene glycol monoethyl ether; alcoholic solvents such as methanol, ethanol, iso- or n-propanol, iso- or n-butanol, and amyl alcohol; ether ester solvents such as ethylene glycol monomethyl ether acetate, ethylene glycol monoethyl ether acetate, propylene glycol monomethyl ether acetate, dipropylene glycol monomethyl ether acetate, 3-methoxybutyl ether acetate, and ethoxyethyl ether propionate; BTX solvents such as benzene, toluene, and xylene; aliphatic hydrocarbon solvents such as hexane, heptane, octane, and cyclohexane; terpene hydrocarbon oils such as turpentine, D-limonene, and pinene; mineral spirits, Swasol #310 (Cosmo Matsuyama Oil Co., Ltd.), Solvesso #100 (Exxon Chemical Company), and other paraffin solvents; halogenated aliphatic hydrocarbon solvents such as carbon tetrachloride, chloroform, trichloroethylene, methylene chloride, and 1,2-dichloroethane; halogenated aromatic hydrocarbon solvents such as chlorobenzene; carbitol solvents; aniline; triethylamine; pyridine; acetic acid; acetonitrile; carbon disulfide; N,N-dimethylformamide; N,N-dimethylacetamide (DMAc); N-methylpyrrolidone; dimethyl sulfoxide; and water. These solvents may be used singly or in a combination of two or more as a solvent mixture.

Of these solvents, ketones and ether ester solvents, in particular, propylene glycol monomethyl ether acetate and cyclohexanone are preferable, for example, in view of their higher ability to dissolve the compound I and the cross-linking agent.

The content of the solvent is preferably from 50 to 99 parts by mass, and more preferably from 70 to 95 parts by mass, in 100 parts by mass of the composition.

Polymerization initiators can be classified into photoinitiators, which generate active species upon irradiation with active energy rays, and thermal initiators, which generate active species upon heating. Examples of the photoinitiators include photo-radical polymerization initiators, photo-acid generators, and photo-base generators. Examples of the thermal initiators include thermal radical polymerization initiators, thermal acid generators, and thermal base generators.

The composition of the present invention may contain an acid generator as the polymerization initiator in order to accelerate the curing reaction of the cross-linking agent. The acid generator can be any compound that can generate an acid under predetermined conditions, and examples thereof include onium salts such as sulfonium salts, iodonium salts, and ammonium salts.

Specific examples of the acid generator include onium salts such as tetramethylammonium trifluoromethanesulfonate, tetramethylammonium nonafluorobutanesulfonate, triethylammonium nonafluorobutanesulfonate, pyridinium nonafluorobutanesulfonate, triethylammonium camphorsulfonate, pyridinium camphorsulfonate, tetra-n-butylammonium nonafluorobutanesulfonate, tetraphenylammonium nonafluorobutanesulfonate, tetramethylammonium p-toluenesulfonate, diphenyliodonium trifluoromethanesulfonate, (p-tert-butoxyphenyl)phenyliodonium trifluoromethanesulfonate, diphenyliodonium p-toluenesulfonate, (p-tert-butoxyphenyl)phenyliodonium p-toluenesulfonate, triphenylsulfonium trifluoromethanesulfonate, (p-tert-butoxyphenyl)diphenylsulfonium trifluoromethanesulfonate, bis(p-tert-butoxyphenyl)phenylsulfonium trifluoromethanesulfonate, tris(p-tert-butoxyphenyl)sulfonium trifluoromethanesulfonate, triphenylsulfonium p-toluenesulfonate, (p-tert-butoxyphenyl)diphenylsulfonium p-toluenesulfonate, bis(p-tert-butoxyphenyl)phenylsulfonium p-toluenesulfonate, tris(p-tert-butoxyphenyl)sulfonium p-toluenesulfonate, triphenylsulfonium nonafluorobutanesulfonate, triphenylsulfonium butanesulfonate, trimethylsulfonium trifluoromethanesulfonate, trimethylsulfonium p-toluenesulfonate, cyclohexylmethyl(2-oxocyclohexyl)sulfonium trifluoromethanesulfonate, cyclohexylmethyl(2-oxocyclohexyl)sulfonium p-toluenesulfonate, dimethylphenylsulfonium trifluoromethanesulfonate, dimethylphenylsulfonium p-toluenesulfonate, dicyclohexylphenylsulfonium trifluoromethanesulfonate, dicyclohexylphenylsulfonium p-toluenesulfonate, trinaphthylsulfonium trifluoromethanesulfonate, cyclohexylmethyl(2-oxocyclohexyl)sulfonium trifluoromethanesulfonate, (2-norbornyl)methyl(2-oxocyclohexyl)sulfonium trifluoromethanesulfonate, ethylenebis[methyl(2-oxocyclopentyl)sulfonium trifluoromethanesulfonate], and 1,2'-naphthylcarbonylmethyltetrahydrothiophenium triflate.

A thermal acid generator, which generates an acid upon exposure to heat, is particularly preferable in view of giving good curability.

The content of the acid generator is preferably 50 to 150 parts by mass, and more preferably 80 to 120 parts by mass, per 100 parts by mass of the cross-linking agent. When the content of the acid generator is within the above-described range, the composition has excellent curability.

In a case where the compound I has a carbon-carbon double bond, the composition of the present invention may contain a radical polymerization initiator as the polymerization initiator. Any of photo-radical polymerization initiators and thermal radical polymerization initiators can be used as the radical polymerization initiator.

Examples of the photo-radical polymerization initiators include: benzoins such as benzoin, benzoin methyl ether, benzoin propyl ether, and benzoin butyl ether; benzyl ketals such as benzyl dimethyl ketal; acetophenones such as acetophenone, 2,2-dimethoxy-2-phenylacetophenone, 2,2-diethoxy-2-phenylacetophenone, 1-benzyl-1-dimethylamino-1-(4'-morpholinobenzoyl)propane, 2-morpholyl-2-(4'-methylmercapto)benzoylpropane, 2-methyl-1-[4-(methylthio)phenyl]-2-morpholino-propan-1-one, 1-hydroxycyclohexylphenyl ketone, 1-hydroxy-1-benzoylcyclohexane, 2-hydroxy-2-benzoylpropane, 2-hydroxy-2-(4'-isopropyl)benzoylpropane, N,N-dimethylaminoacetophenone, 1,1-dichloroacetophenone, 4-butylbenzoyltrichloromethane, and 4-phenoxybenzoyldichloromethane; anthraquinones such as 2-methylanthraquinone, 1-chloroanthraquinone, and 2-amylanthraquinone; thioxanthones such as 2,4-dimethylthioxanthone, 2,4-diethylthioxanthone, 2-chlorothioxanthone, and 2,4-diisopropylthioxanthone; ketals such as acetophenone dimethyl ketal and benzyl dimethyl ketal; benzophenones such as benzophenone, methylbenzophenone, 4,4'-dichlorobenzophenone, 4,4'-bisdiethylaminobenzophenone, Michler's ketone, and 4-benzoyl-4'-methyldiphenyl sulfide; oxides such as 2,4,6-trimethylbenzoyldiphenylphosphine oxide and bis(2,4,6-trimethylbenzoyl)-phenylphosphine oxide; carbazoles such as 3-(2-methyl-2-morpholinopropionyl)-9-methylcarbazole; α-dicarbonyls such as benzyl and methyl benzoylformate; oxime esters such as compounds disclosed in JP 2000-80068A, JP 2001-233842A, JP 2005-97141A, JP 2006-516246T, JP 3860170B, JP 3798008B, WO 2006/018973, JP 2011-132215A, and WO 2015/152153; triazines such as p-methoxyphenyl-2,4-bis(trichloromethyl)-s-triazine, 2-methyl-4,6-bis(trichloromethyl)-s-triazine, 2-phenyl-4,6-bis(trichloromethyl)-s-triazine, 2-naphthyl-4,6-bis(trichloromethyl)-s-triazine, and 2-(p-butoxystyryl)-s-triazine; and benzoyl peroxide, 2,2'-azobisisobutyronitrile, ethyl anthraquinone, 1,7-bis(9'-acridinyl)heptane, thioxanthone, 1-chlor-4-propoxythioxanthone, isopropylthioxanthone, diethylthioxanthone, benzophenone, phenyl biphenyl ketone, 4-benzoyl-4'-methyldiphenyl sulfide, 2-(p-butoxystyryl)-5-trichloromethyl-1,3,4-oxadiazole, 9-phenylacridine, 9,10-dimethyl benzphenazine, benzophenone/Michler's ketone, hexaarylbiimidazole/mercaptobenzimidazole, and thioxanthone/amine.

Examples of the thermal radical polymerization initiators include: peroxides such as benzoyl peroxide, 2,4-dichlorobenzoyl peroxide, 1,1-di(t-butylperoxy)-3,3,5-trimethylcyclohexane, 4,4-di(t-butylperoxy)butyl valerate, and dicumyl peroxide; azo compounds such as 2,2'-azobisisobutyronitrile; and tetramethylthiuram disulfide.

Examples of the photo-base generators include carbamate compounds, α-amino ketone compounds, quaternary ammonium compounds, O-acyloxime compounds, and aminocyclopropenone compounds.

Examples of the carbamate compounds include 1-(2-anthraquinonyl)ethyl 1-piperidinecarboxylate, 1-(2-anthraquinonyl)ethyl 1H-2-ethylimidazole-1-carboxylate, 9-anthrylmethyl 1-piperidinecarboxylate, 9-anthrylmethyl N,N-diethylcarbamate, 9-anthrylmethyl N-propylcarbamate, 9-anthrylmethyl N-cyclohexylcarbamate, 9-anthrylmethyl 1H-imidazole-1-carboxylate, 9-anthrylmethyl N,N-dioctylcarbamate, 9-anthrylmethyl 1-(4-hydroxypiperidine)carboxylate, 1-pyrenylmethyl 1-piperidinecarboxylate, bis[1-(2-anthraquinonyl)ethyl] 1,6-hexanediylbiscarbamate, bis(9-anthrylmethyl) 1,6-hexanediylbiscarbamate, and the like.

Examples of the α-amino ketone compounds include 1-phenyl-2-(4-morpholinobenzoyl)-2-dimethylaminobutane, and 2-(4-methylthiobenzoyl)-2-morpholinopropane.

Examples of the quaternary ammonium compounds as photo-base generators include 1-(4-phenylthiophenacyl)-1-azonia-4-azabicyclo[2,2,2]octane tetraphenylborate, 5-(4-phenylthiophenacyl)-1-aza-5-azoniabicyclo[4,3,0]-5-nonene tetraphenylborate, and 8-(4-phenylthiophenacyl)-1-aza-8-azoniabicyclo[5,4,0]-7-undecene tetraphenylborate.

Examples of the aminocyclopropenone compounds as photo-base generators include 2-diethylamino-3-phenylcyclopropenone, 2-diethylamino-3-(1-naphthyl)cyclopropenone, 2-pyrrolidinyl-3-phenylcyclopropenone, 2-imidazolyl-3-phenylcyclopropenone, and 2-isopropylamino-3-phenylcyclopropenone.

Examples of the thermal base generators include: carbamate derivatives such as 2-(4-biphenyl)-2-propylcarbamate and 1,1-dimethyl-2-cyanoethylcarbamate; urea and urea derivatives such as N,N,N'-trimethylurea; dihydropyridine derivatives such as 1,4-dihydronicotinamide; and salts constituted by an acid and a base, such as organic and inorganic salts.

The compound and the composition of the present invention have excellent heat resistance, and can therefore be suitably applied to electronic component, such as semiconductor encapsulants, circuit boards, build-up films, build-up PCBs, resists for semiconductor processing, hard masks for semiconductor processing, and multilayer flexible films. Examples of the electronic component include: discretes (individual semiconductors), which is a single element having a single function, such as transistors and diodes; integrated circuits (ICs), which has a single chip and elements with multiple functions mounted thereon; and CPUs such as memories, microprocessors (MPUs), and logic ICs. Electronic components in which the composition of the present invention is used can be suitably used in various applications, and examples thereof include, but not limited to, industrial machine parts, general machine parts, parts for automobiles, railroads, vehicles, and the like, aerospace-related parts, electronic and electrical parts, building materials, container and packaging members, household goods, sports and leisure goods, and casing members for wind power generation.

As shown in Examples, which will be described later, the composition of the present invention has excellent heat resistance and excellent solvent resistance, and can therefore be suitably used for materials for electronic components. In particular, as shown in Examples, the composition is excellent in the filling property and planarization, and can therefore be suitably used for materials for forming film in semiconductor processing. The materials for forming film in semiconductor processing are materials necessary for forming film in semiconductor processing, and include materials for forming semiconductor components and materials for forming components for semiconductor processing.

The materials for forming semiconductor components include materials suitable for applications of the respective components. Examples of thereof include a material for forming insulating film, a material for forming barrier film, a material for encapsulant, and a material for gap filler.

The materials for forming components for semiconductor processing include materials suitable for applications of the respective components. Examples of thereof include a material for forming underlayer film, a material for photoresist, a material for forming antireflection film, and a material for forming interlayer film.

The films in semiconductor processing are films that are used to produce semiconductors. Specifically, the term "film in semiconductor processing" collectively refers to solid layers obtained by applying a material for forming film in semiconductor processing and removing a solvent therefrom, and cured products obtained by curing the solid layer through, for example, a polymerization reaction.

The films in semiconductor processing encompass semiconductor components and components for semiconductor processing. The semiconductor component means a component that remains as a permanent film in the semiconductor device. The component for semiconductor processing means a component that is used as sacrificial film in the process of producing semiconductors but does not remain in the semiconductor device.

Examples of the semiconductor components include insulation films, barrier films, encapsulants, and gap fillers.

Examples of the components for semiconductor processing include photoresists, interlayer films, underlayer films, and antireflection films.

The photoresists, the interlayer films, and the underlayer films are components for semiconductor processing that are used in order to obtain a pattern of good quality. They can be used in the form of a multilayer resist constituted by an underlayer film, an interlayer film, and a photoresist layered in this order on a substrate as a processing target, such as a silicon wafer.

The composition for an electronic component of the present invention encompasses those included in the cured product of the present invention and those not included in the cured product because it is used during the producing process and removed after use as a sacrificial film.

The composition for an electronic component of the present invention may be for an underlayer film in a multilayer resist, for example.

One exemplary overview of a semiconductor processing involving use of a multilayer resist is shown below.

A common example of a multilayer resist is based on the three-layer resist method including forming a single photoresist layer and two layers thereunder. Specifically, the method may be as follows.

First, a material for forming underlayer film is applied to a substrate as a processing target, such as a silicon wafer, and is heated to form an underlayer film. Then, an interlayer film is formed on the underlayer film. A material for photoresist is applied thereon, followed by forming a pattern through exposure and development. The resulting pattern is used as a mask to etch the interlayer film and the underlayer film in this order under appropriate dry etching conditions. Then, the resulting underlayer film pattern is used as a mask to etch the substrate as a processing target under appropriate dry etching conditions, and the remaining mask is subjected to ashing to obtain a substrate with the desired structure.

The material for forming underlayer film needs to have heat resistance and solvent resistance that will prevent deformation of the underlayer film by heat or solvent during the formation of an interlayer film or a photoresist layer, and also needs to have etching resistance to ensure precise transfer by etching. In addition, the material for forming underlayer film further needs to have the capability to fill unevenness of substrates and the capability to form planar films. The composition of the present invention is particularly suitably used for such a material for forming underlayer film.

The compound and the composition of the present invention is excellent in the capability to fill unevenness of substrates. Accordingly, the compound and composition can also be used for a material for gap filler to be filled into recesses formed on substrates (filling and planarizing film). The compound and the composition can also be used for an insulating material to be filled into recesses formed on substrates (filling insulation film), a barrier material, a semiconductor resist material for a component other than the underlayer film described above, or an insulation film for through-silicon vias, for example.

### C. Cured Product

The cured product of the present invention is a cured product obtained by curing the above-described composition. The cured product is preferably a thermally cured product in view of excellent heat resistance and excellent solvent resistance.

### D. Method for Producing Cured Product

The method for producing a cured product includes the step of curing the composition.

In the case of thermal curing, curing can be performed through heating using a heat plate such as a hot plate, an atmospheric oven, an inert gas oven, a vacuum oven, or a convection oven.

The heating temperature during thermal curing is preferably 150 to 500°C, more preferably 200 to 450°C, and even more preferably 250 to 400°C. The heating temperature within the above-mentioned range can result in a cured product having excellent heat resistance.

The curing time is not particularly limited, but is preferably 30 seconds to 60 minutes, and more preferably 1 to 30 minutes, in view of improving the productivity.

### E. Method for Producing Electronic Component

The method for producing an electronic component includes the step of curing the composition and then the step of removing the cured product. For example, the method includes: the step of forming a layer of the composition of the present invention on a substrate; the step of curing the layer to form a cured film; and then the step of patterning the cured film by etching. When the present invention is applied to an underlayer film for producing a semiconductor, a specific example of the process is, for example, as follows.

A method for producing an electronic component, including:
the step of forming an underlayer film by applying a layer of the composition of the present invention to a surface of a substrate as a processing target and then curing the layer;
the step of applying a photoresist layer to the underlayer film;
the step of forming a resist pattern, by placing a mask on the photoresist layer, exposing the photoresist layer to energy rays through the mask, and then developing the exposed photoresist layer; and
the step of partially removing the underlayer film by transferring the pattern to the underlayer film.

By the partial removal of the underlayer film as described above, corresponding portions of the substrate as a processing target are exposed.

An interlayer film may be formed between the underlayer film and the photoresist. As the interlayer film, a silicon-containing layer, a hard mask layer, or any other conventionally known interlayer films may be used. For removal of the underlayer film through patterning, known plasma etching can be used.

### Examples

Hereinafter, the present invention will be described more specifically by way of examples and comparative examples, but the present invention is not limited to the examples below.

### Example 1-1

5.0 g (34 mmol) of 6-hydroxy-1-indanone, 2.2 g (18 mmol) of 4-hydroxybenzaldehyde, 5.5 g (72 mmol) of ammonium acetate, and 25 g of ethanol were placed in a reaction flask equipped with a reflux tube, and were refluxed while stirring. After 12.5 hours, the mixture was returned to room temperature, 17 g of ion-exchange water was added, and the precipitate was collected by filtration. The filter cake was washed with ethanol/ion-exchange water, and dried under reduced pressure at 40°C to obtain Compound A1 as a pale yellow powder.
Actual yield: 1.4 g (percent yield: 21%)
¹H-NMR (DMSO-d₆) δ/ppm: 3.78 (s, 4H), 6.82 (d, 2H), 6.93 (d, 2H), 7.38 (d, 2H), 7.45 (s, 2H), 7.56 (d, 2H), 9.52 (s, 3H)

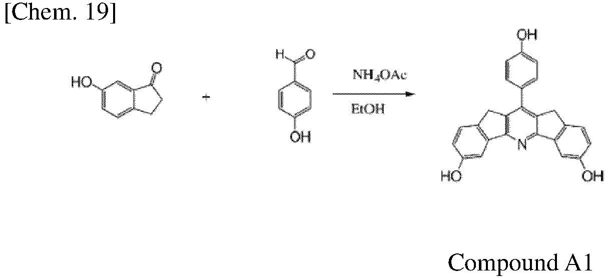

### Example 1-2

23.7 g (160 mmol) of 5-hydroxy-1-indanone, 9.8 g (80 mmol) of 4-hydroxybenzaldehyde, 28.4 g (368 mmol) of ammonium acetate, and 130 g of ethanol were placed in a reaction flask equipped with a reflux tube, and were refluxed while stirring. After 6.5 hours, the mixture was returned to room temperature, and the precipitate was collected by filtration. The filter cake was washed with ethanol/ion-exchange water, and dried under reduced pressure at 40°C to obtain Compound A2 as a pale yellow powder.
Actual yield: 11.4 g (percent yield: 38%)
¹H-NMR (DMSO-d₆) δ/ppm: 3.79 (s, 4H), 6.87 (d, 2H), 6.93 (d, 2H), 6.98 (s, 2H), 7.55 (d, 2H), 7.83 (d, 2H), 9.66 (s, 3H)

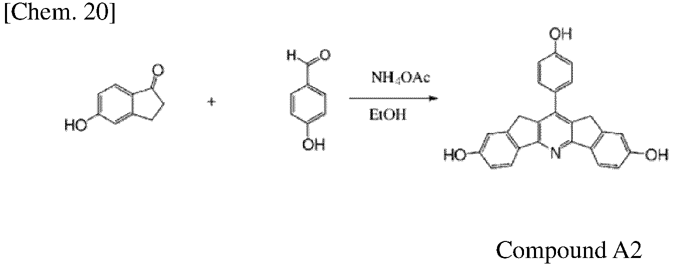

### Example 1-3

7.6 g (58 mmol) of 1-indanone, 3.5 g (29 mmol) of 4-hydroxybenzaldehyde, 8.8 g (12 mmol) of ammonium acetate, and 50 g of ethanol were placed in a reaction flask equipped with a reflux tube, and were refluxed while stirring. After 6 hours, the mixture was returned to room temperature, and the precipitate was collected by filtration. The filter cake was washed with ethanol/ion-exchange water, and dried under reduced pressure at 60°C to obtain Compound A3 as a yellow powder.
Actual yield: 3.0 g (percent yield: 30%)
¹H-NMR (DMSO-d₆) δ/ppm: 3.92 (s, 4H), 6.98 (d, 2H), 7.41-7.60 (m, 4H), 7.61-7.64 (m, 4H), 8.09 (d, 2H), 9.78 (s, 1H)

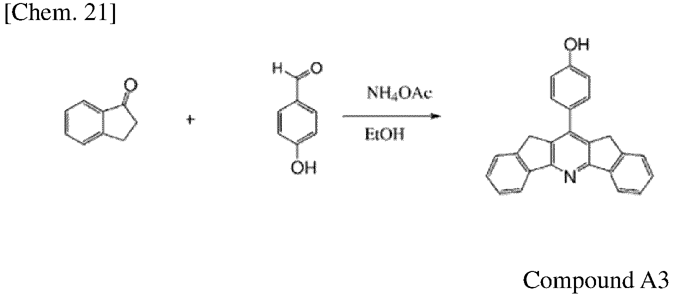

### Example 1-4

30.0 g (227 mmol) of 1-indanone, 15.7 g (114 mmol) of 2,4-dihydroxybenzaldehyde, 70.0 g (908 mmol) of ammonium acetate, and 197 g of ethanol were placed in a reaction flask equipped with a reflux tube, and were refluxed while stirring. After 4.5 hours, the mixture was returned to room temperature, and the precipitate was collected by filtration. The filter cake was washed with ethanol/ion-exchange water and then with toluene/DMF, and dried under reduced pressure at 60°C to obtain Compound A4 as a pale red powder.
Actual yield: 5.4 g (percent yield: 14%)
¹H-NMR (DMSO-d₆) δ/ppm: 3.83 (d, 4H), 6.42 (d, 1H), 6.54 (d, 1H), 7.27 (d, 1H), 7.41-7.51 (m, 4H), 7.62 (d, 2H), 8.09 (d, 2H), 9.55 (s, 1H), 9.64 (s, 1H)

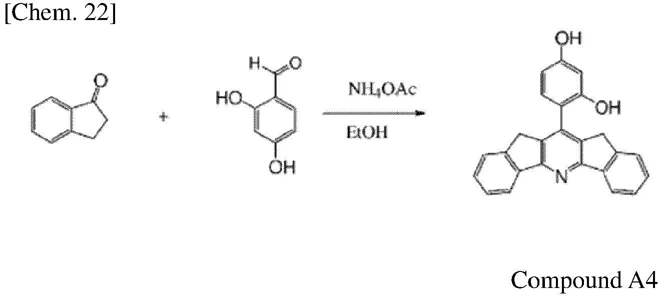

### Example 1-5

### (Step 1)

19.8 g (103 mmol) of 5,6-dimethoxy indanone, 10.0 g (52 mmol) of 2-fluorenecarboxyaldehyde, 15.9 g (206 mmol) of ammonium acetate, and 139 g of ethanol were placed in a reaction flask equipped with a reflux tube, and were refluxed while stirring. After 3 hours, the mixture was returned to room temperature, and the precipitate was collected by filtration. The filter cake was washed with ethanol/acetone and then with NMP, and dried under reduced pressure at 100°C to obtain Compound A5 as a yellow powder.
Actual yield: 5.1 g (percent yield: 23%)
¹H-NMR (DMSO-d₆) δ/ppm: 3.84 (s, 10H), 3.95 (s, 6H), 4.06 (s, 2H), 7.25 (s, 2H), 7.39 (m, 1H), 7.46 (t, 1H), 7.62 (s, 2H), 7.67 (d, 1H), 7.80 (d, 1H), 8.00 (s, 1H), 8.02 (d, 1H), 8.09 (d, 1H)

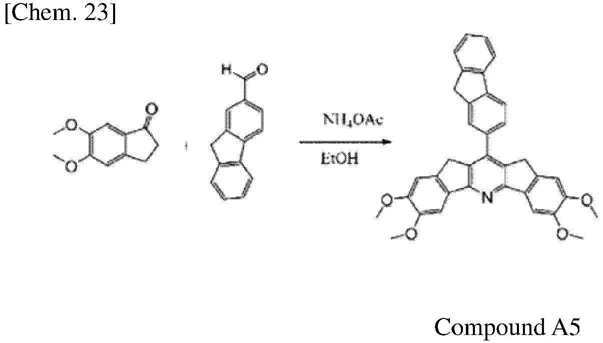

### (Step 2)

4.0 g (7.4 mmol) of compound A5, 12.0 g (60 mmol) of 1-dodecanethiol, 4.8 g (118 mmol) of NaOH, and 40 g of NMP were placed in a reaction flask equipped with a reflux tube, and stirred at 120°C. After 2 hours, the mixture was cooled, and 20 g of ion-exchange water was added. The mixture was neutralized with dilute hydrochloric acid, and the precipitate was collected by filtration. The filter cake was washed with methanol and then with DMF, and dried under reduced pressure at 100°C to obtain Compound A6 as a yellow powder.
Actual yield: 0.2 g (percent yield: 14%)
¹H-NMR (DMSO-d6) δ/ppm: 3.77 (s, 4H), 3.97 (s, 6H), 4.05 (s, 2H), 7.01 (s, 2H), 7.36-7.48 (m, 2H), 7.59 (s, 2H), 7.65 (d, 1H), 7.74 (d, 1H), 7.95 (s, 1H), 8.01 (d, 1H), 8.07 (d, 1H), 9.23 (s, 2H)

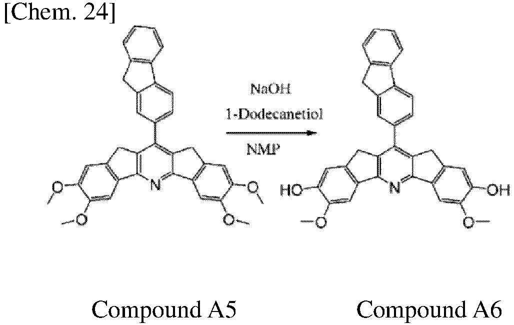

### Example 1-6

### (Step 1)

8.1 g (61 mmol) of 1-indanone, 7.0 g (30 mmol) of 1-pyrenecarboxyaldehyde, 9.8 g (127 mmol) of ammonium acetate, and 70 g of ethanol were placed in a reaction flask equipped with a reflux tube, and were refluxed while stirring. After 7 hours, the mixture was returned to room temperature, and the precipitate was collected by filtration. The filter cake was washed with THF/acetone, and dried under reduced pressure at 40°C to obtain Compound A7 as a yellow powder.
Actual yield: 3.0 g (percent yield: 22%)
¹H-NMR (CDCl₃) δ/ppm: 3.65 (q, 4H), 7.36-7.42 (m, 4H), 7.53 (t, 2H), 7.72 (d, 1H), 7.98 (d, 1H), 8.05 (t, 2H), 8.19 (d, 3H), 8.29 (d, 1H), 8.34-8.39 (m, 3H)

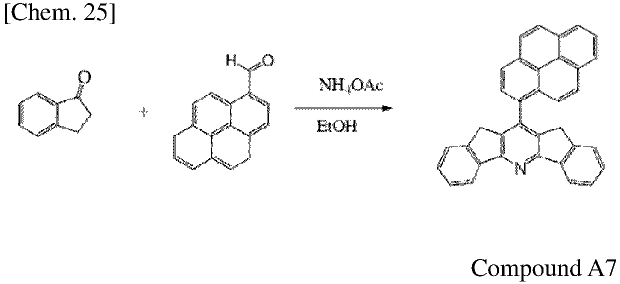

### (Step 2)

2.8 g (6.1 mmol) of compound A7 and 20 g of DMF were placed in a reaction flask equipped with a reflux tube, and stirred at room temperature under nitrogen flow. 6.5 g (78 mmol) of 48% aqueous sodium hydroxide solution was added, and then 9.3 g (79 mmol) of 3-bromo-1-propyne was added dropwise, followed by stirring the mixture at 60°C for 12 hours. Then, 50 g of ion-exchange water was placed in a beaker, and the reaction solution was added thereto. Methanol was also added thereto, and the precipitate was collected by filtration. The filter cake was washed with ion-exchange water/methanol, and dried under reduced pressure at 40°C to obtain Compound A8 as a yellow powder.
Actual yield: 0.6 g (percent yield: 16%)
¹H-NMR (DMSO-d₆) δ/ppm: 1.80 (d, 2H), 1.92 (d, 2H), 2.41-2.47 (m, 6H), 2.72 (d, 2H), 7.45-7.60 (m, 6H), 8.06-8.20 (m, 5H), 8.36-8.48 (m, 5H), 8.55 (d, 1H)

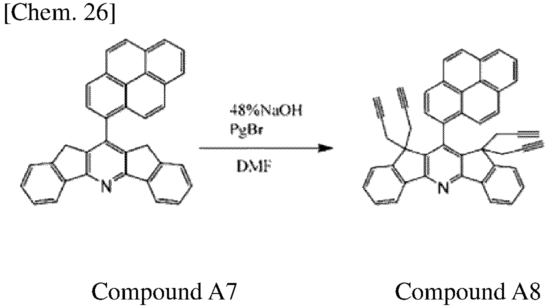

### Example 1-7

### (Step 1)

7.6 g (58 mmol) of 1-indanone, 6.0 g (31 mmol) of 2-fluorenecarboxyaldehyde, 8.6 g (111 mmol) of ammonium acetate, and 60 g of ethanol were placed in a reaction flask equipped with a reflux tube, and were refluxed while stirring. After 8 hours, the mixture was returned to room temperature, and the precipitate was collected by filtration. The filter cake was washed with ion-exchange water/ethanol and then with DMF/acetone, and dried under reduced pressure at 40°C to obtain Compound A9 as a pale yellow powder.
Actual yield: 3.9 g (percent yield: 30%)
¹H-NMR (CDCl₃) δ/ppm: 3.88 (s, 4H), 4.04 (s, 2H), 7.35-7.63 (m,10H), 7.73 (s, 1H), 7.88 (d, 1H), 7.96 (d, 1H), 8.30 (d, 2H)

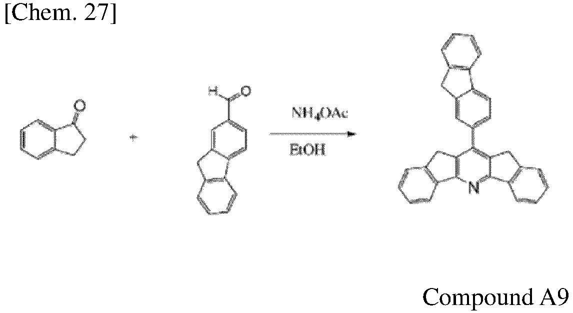

### (Step 2)

3.4 g (8.1 mmol) of compound A9, 0.8 g (2.4 mmol) of tetrabutylammonium bromide, and 15 g of cyclopentyl methyl ether were placed in a reaction flask equipped with a reflux tube, and stirred at room temperature under nitrogen flow. 11.9 g (143 mmol) of 48% aqueous sodium hydroxide solution and 10 g of ion-exchange water were added, and 13.4 g (113 mmol) of 3-bromo-1-propyne was added dropwise, followed by stirring the mixture at 70°C for 13 hours. Then, 50 g of hexane was placed in a beaker, the reaction solution was added thereto, and the precipitate was collected by filtration. The filter cake was dissolved in 60 g of methylene chloride, followed by washing with water three times, and the solvent was evaporated off. The residue was crystallized from methyl tert-butyl ether, followed by filtration. The filter cake was dried under reduced pressure at 40°C to obtain Compound A10 as a yellow powder.
Actual yield: 1.5 g (percent yield: 28%)
¹H-NMR (CDCl₃) δ/ppm: 1.62 (q, 4H), 1.92 (s, 2H), 2.42 (dd, 4H), 2.82 (d, 4H), 2.96 (s, 4H), 7.43-7.54 (m, 6H), 7.60 (d, 2H), 7.70 (dd, 2H), 7.85 (d, 1H), 7.93 (d, 1H), 8.02 (s, 1H), 8.25 (d, 2H)

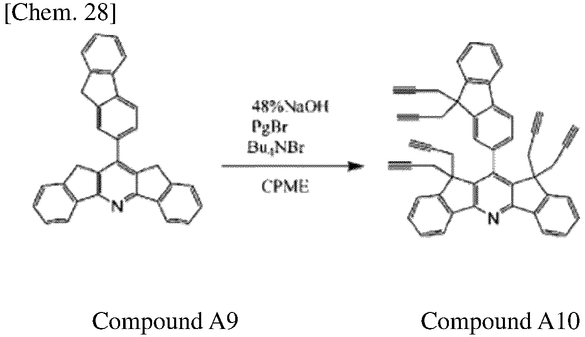

### Example 1-8

1.7 g (3.7 mmol) of compound A1 and 31 g of DMF were placed in a reaction flask equipped with a reflux tube, and stirred at room temperature under nitrogen flow. 7.9 g (94 mmol) of 48% aqueous sodium hydroxide solution was added, and 10.6 g (89 mmol) of 3-bromo-1-propyne was added dropwise, followed by stirring at 65°C for 17.5 hours. Then, 50 g of ion-exchange water was placed in a beaker, the reaction solution was added thereto, and the precipitate was collected by filtration. The filter cake was washed with ion-exchange water/ethanol, and dried under reduced pressure at 40°C to obtain Compound A11 as a yellow powder.
Actual yield: 0.7 g (percent yield: 32%)
¹H-NMR (DMSO-d₆) δ/ppm: 2.20 (d, 4H), 2.35 (s, 4H), 2.81 (d, 4H), 3.65 (s, 3H), 4.95 (s, 6H), 7.07 (d, 2H), 7.25 (d, 2H), 7.56-7.58 (m, 6H)

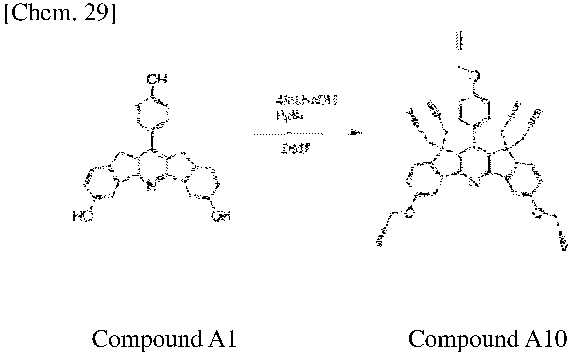

### Example 1-9

3.1 g (8.0 mmol) of compound A2, 4.8 g (35 mmol) of potassium carbonate, and 44 g of DMF were placed in a reaction flask equipped with a reflux tube, and stirred at room temperature under nitrogen flow. Then, 3.7 g (31 mmol) of 3-bromo-1-propyne was added dropwise, and the mixture was stirred at 60°C for 7 hours. Then, 196 g of ion-exchange water was placed in a beaker, the reaction solution was added thereto, and the precipitate was collected by filtration. The filter cake was washed with methanol, and dried under reduced pressure at 40°C to obtain Compound A12 as a yellow powder.
Actual yield: 3.3 g (percent yield: 83%)
¹H-NMR (DMSO-d₆) δ/ppm: 3.60 (s, 2H), 3.65 (s, 1H), 3.88 (s, 4H), 4.87 (s, 4H), 4.93 (s, 2H), 7.11 (d, 2H), 7.18 (d, 2H), 7.24 (s, 2H), 7.75 (d, 2H), 7.98 (d, 2H)

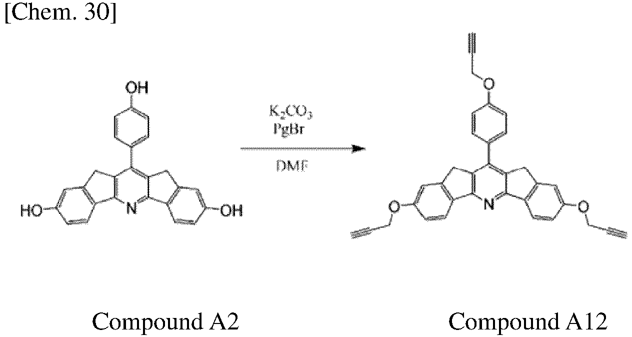

### Example 1-10

3.1 g (8.0 mmol) of compound A2, 0.8 g (2.4 mmol) of tetrabutylammonium bromide, and 15 g of cyclopentyl methyl ether were placed in a reaction flask equipped with a reflux tube, and stirred at room temperature under nitrogen flow. 12.0 g (144 mmol) of 48% aqueous sodium hydroxide solution and 5 g of ion-exchange water were added, and 7.3 g (61 mmol) of 3-bromo-1-propyne was added dropwise, followed by stirring at 55°C for 6.5 hours. Then, 16 g methanol and 10 g of ion-exchange water were added, and the precipitate was collected by filtration. The filter cake was washed with methanol/ion-exchange water, and dried under reduced pressure 50°C to obtain Compound A13 as a yellow powder.
Actual yield: 3.1 g (percent yield: 61%)
¹H-NMR (DMSO-d₆) 2.19 (d, 4H), 2.35 (s, 4H), 2.80 (d, 4H), 3.61 (s, 2H), 3.65 (s, 1H), 4.88 (s, 4H), 4.94 (s, 2H), 7.10 (d, 2H), 7.25 (d, 2H), 7.30 (s, 2H), 7.52 (d, 2H), 7.89 (d, 2H)

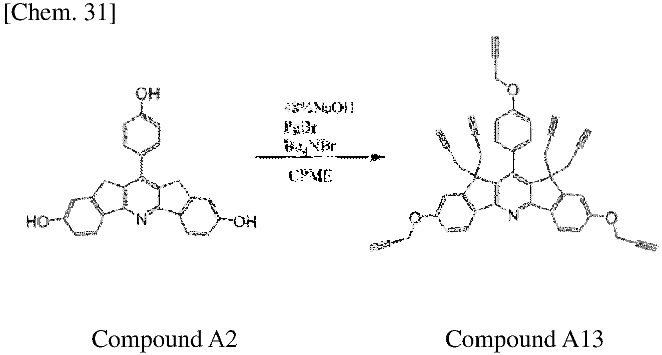

### Example 1-11

1.0 g (2.9 mmol) of compound A3 and 8 g of DMF were placed in a reaction flask equipped with a reflux tube, and stirred at room temperature under nitrogen flow. 1.3 g (15 mmol) of 48% aqueous sodium hydroxide solution was added, and 1.8 g (15 mmol) of 3-bromo-1-propyne was added dropwise, followed by stirring for 3 hours. Then, 62 g of ion-exchange water was placed in a beaker, and the reaction solution was added thereto. The precipitate was collected by filtration, and the filter cake was washed with ion-exchange water/methanol, and dried under reduced pressure at 60°C to obtain Compound A14 as a pale orange powder.
Actual yield: 1.3 g (percent yield: 86%)
¹H-NMR (DMSO-d₆) δ/ppm: 2.23 (d, 2H), 2.27 (d, 2H), 2.35 (t, 4H), 2.85 (d, 2H), 2.89 (d, 2H), 3.67 (t, 1H), 4.96 (d, 2H), 7.28 (d, 2H), 7.46-7.57 (m, 6H), 7.68 (d, 2H), 8.02 (d, 2H)

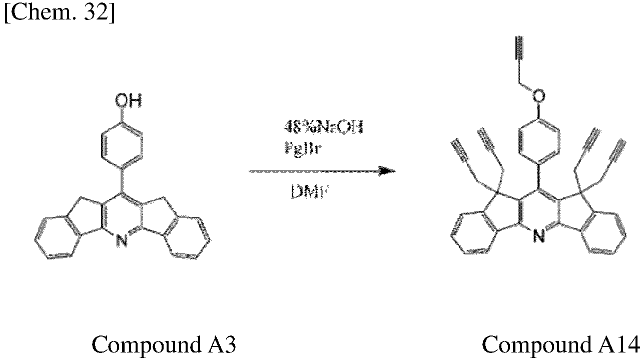

### Example 1-12

5.0 g (14 mmol) of compound A4 and 33 g of DMF were placed in a reaction flask equipped with a reflux tube, and stirred at room temperature under nitrogen flow. 7.2 g (87 mmol) of 48% aqueous sodium hydroxide solution was added, and 10.3 g (87 mmol) of 3-bromo-1-propyne was added dropwise, followed by stirring at 60 to 80°C for 4 hours. The salts in the reaction system were removed by filtration. 160 g of ion-exchange water was placed in a beaker, and the reaction solution was added thereto. The precipitate was collected by filtration, and the filter cake was washed with ion-exchange water/methanol, and dried under reduced pressure at 60°C to obtain Compound A15 as a pale orange powder. Actual yield: 1.3 g (percent yield: 86%)
¹H-NMR (DMSO-d₆) δ/ppm: 2.23 (d, 2H), 2.27 (d, 2H), 2.35 (t, 4H), 2.85 (d, 2H), 2.89 (d, 2H), 3.53 (t, 1H), 3.68 (t, 1H), 4.84 (d, 2H), 4.93 (d, 2H), 6.84 (d, 1H), 6.96 (d, 2H), 7.42-7.54 (m, 5H), 7.62 (d, 2H), 8.12 (d, 2H)

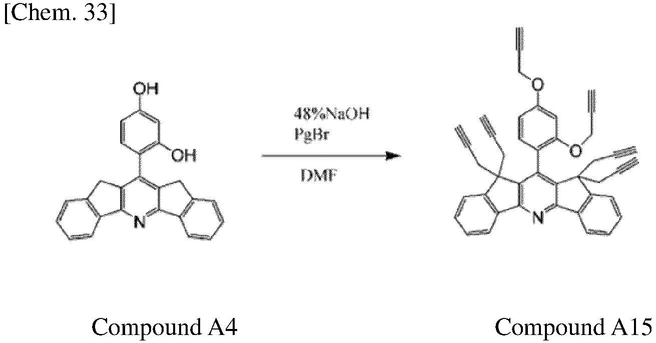

### Example 1-13

### (Step 1)

29.0 g (219 mmol) of 1-indanone, 24.5 g (110 mmol) of 3-formyl-N-ethylcarbazole, 67.7 g (878 mmol) of ammonium acetate, and 246.1 g of ethanol were placed in a reaction flask equipped with a reflux tube, and were refluxed while stirring. After 1 hour, the mixture was returned to room temperature, and the precipitate was collected by filtration. The filter cake was washed with acetone and then with THF, and dried under reduced pressure at 60°C to obtain Compound A16 as a pale red powder.
Actual yield: 9.8 g (percent yield: 20%)
¹H-NMR (DMSO-d₆) δ/ppm: 1.41 (t, 3H), 4.05 (s, 4H), 4.55 (q, 2H), 7.25 (t, 1H), 7.43-7.54 (m, 5H), 7.62 (d, 2H), 7.68 (d, 1H), 7.79 (d, 1H), 7.89 (d, 1H), 8.14 (d, 2H), 8.31 (d, 1H), 8.66 (d, 1H)

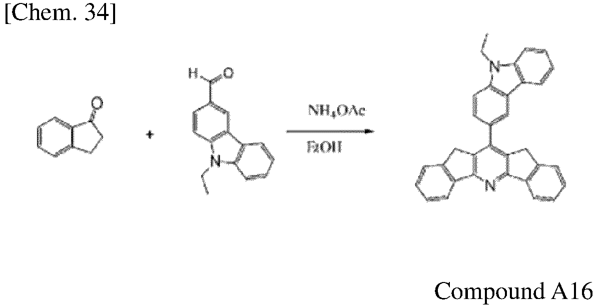

### (Step 2)

4.0 g (8.9 mmol) of compound A16, 0.9 g (2.7 mmol) of tetrabutylammonium bromide, and 21 g of cyclopentyl methyl ether were placed in a reaction flask equipped with a reflux tube, and stirred at room temperature under nitrogen flow. 8.9 g (107 mmol) of 48% aqueous sodium hydroxide solution and 4 g of ion-exchange water were added, and the temperature was raised to 70°C. Then, 6.4 g (54 mmol) of 3-bromo-1-propyne was added dropwise, and the mixture was stirred at 85°C for 2 hours. The mixture was cooled to 30°C, 48 g of ion-exchange water was added, and the precipitate was collected by filtration. The filter cake was washed with methanol/ion-exchange water, and dried under reduced pressure at 60°C to obtain Compound A17 as a pale yellow powder.
Actual yield: 2.8 g (percent yield: 52%)
¹H-NMR (DMSO-d₆) δ/ppm: 1.46 (t, 3H), 2.24 (dd, 4H), 2.38 (t, 2H), 2.44 (t, 2H), 2.80 (dd, 2H), 2.84 (dd, 2H), 4.59 (q, 2H), 7.27 (t, 1H), 7.45-7.58 (m, 5H), 7.65 (d, 2H), 7.70-7.76 (m, 4H), 7.93 (d, 1H), 8.02 (d, 1H), 8.05 (d, 2H), 8.36 (d, 1H)

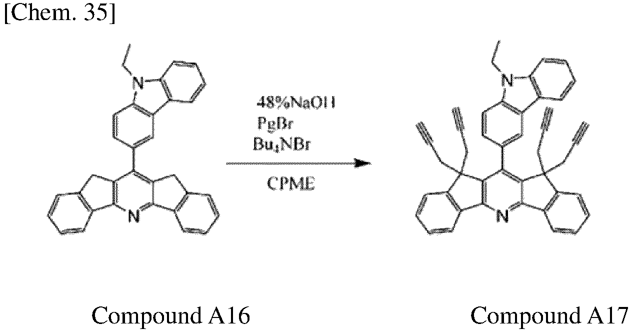

### Example 1-14

2.5 g (4.9 mmol) of compound A6, 0.5 g (1.5 mmol) of tetrabutylammonium bromide, and 25 g of cyclopentyl methyl ether were placed in a reaction flask equipped with a reflux tube, and stirred at room temperature under nitrogen flow. 8.2 g (98 mmol) of 48% aqueous sodium hydroxide solution and 3 g of ion-exchange water were added, and the temperature was raised to 50°C. Then, 7.3 g (61 mmol) of 3-bromo-1-propyne was added dropwise, and the mixture was stirred at 60°C for 3 hours. The mixture was cooled to 30°C, the insoluble matters in the reaction system were removed, and the solvent was evaporated off. Then, 5 g of cyclopentyl methyl ether, 5 g of ion-exchange water, and 15 g of methanol were added, and the precipitate was collected by filtration. The filter cake was washed with methanol/ion-exchange water, and dried under reduced pressure 50°C to obtain Compound A18 as a yellow powder.
Actual yield: 0.5 g (percent yield: 13%)
¹H-NMR (DMSO-d₆) δ/ppm: 2.24 (t, 1H), 2.29 (t, 1H), 2.34 (d, 2H), 2.36 (t, 2H), 2.39 (d, 2H) 2.48 (t, 2H), 2.73-2.81 (m, 4H), 2.90-3.00 (m, 4H), 3.58 (t, 2H), 3.97 (s, 6H), 4.84 (d, 4H), 7.35-7.64 (m, 7H), 7.76 (d, 1H), 7.97 (d, 2H), 8.10 (d, 1H)

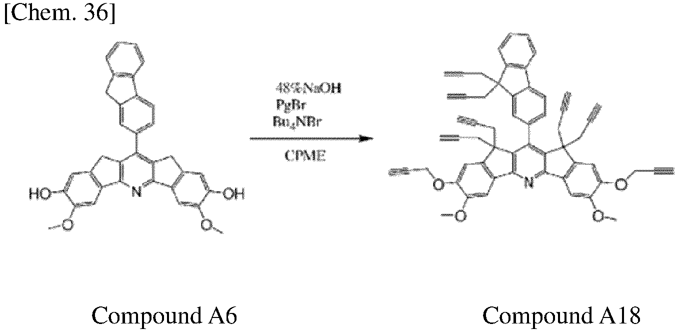

### Example 1-15

### (Step 1)

Compound A29 as a pale yellow powder was obtained in the same synthetic manner as in Example 1-1, except that 6-hydroxy-1-indanone was changed to 5-hydroxy-1-indanone and 4-hydroxybenzaldehyde was changed to 3-hydroxybenzaldehyde.

### (Step 2)

9.0 g (23.7 mmol) of compound A29, 2.3 g (7.1 mmol) of tetrabutylammonium bromide, and 18.0 g of cyclopentyl methyl ether were placed in a reaction flask equipped with a reflux tube, and stirred at room temperature under nitrogen flow. 35.6 g (427 mmol) of 48% aqueous sodium hydroxide solution was added, and the temperature was raised to 60°C. Then, 21.7 g (183 mmol) of 3-bromo-1-propyne was added dropwise, and the mixture was stirred at 60°C for 2 hours. The mixture was cooled to 30°C, and the solvent was evaporated off from the organic layer. Then, 95 g of methanol was added, and the precipitate was collected by filtration. The filter cake was washed with methanol/ion-exchange water, and dried under reduced pressure at 40°C to obtain Compound A22 as a pale yellow powder.
Actual yield: 6.5 g (percent yield: 42.4%)
¹H-NMR (DMSO-d₆) δ/ppm: 2.22-2.24 (m, 2H), 2.26-2.28 (m, 2H), 2.35-2.38 (m, 4H), 2.81-2.82 (m, 2H), 2.85-2.87 (m, 2H), 3.58 (t, 1H), 3.62 (t, 2H), 4.87 (d, 2H), 4.87 (d, 4H), 7.11-7.14 (m, 2H), 7.22-7.29 (m, 3H), 7.58-7.63 (m, 5H)

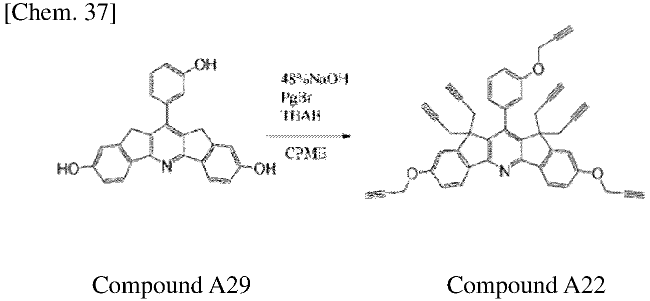

### Example 1-16

### (Step 1)

Compound A30 as a pale yellow powder was obtained in the same synthetic manner as in Example 1-1, except that 4-hydroxybenzaldehyde was changed to 3-hydroxybenzaldehyde,.
¹H-NMR (DMSO-d₆) δ/ppm: 3.79 (s, 4H), 6.93 (dd, 2H), 6.93 (d, 1H), 7.03 (d, 2H), 7.08 (s, 1H), 7.15 (s, 1H), 7.40 (t, 1H), 7.86 (d, 2H) 9.65 (s, 2H), 9.67 (s, 1H)

### (Step 2)

9.0 g (23.7 mmol) of compound A30, 2.3 g (7.1 mmol) of tetrabutylammonium bromide, and 18.0 g of cyclopentyl methyl ether were placed in a reaction flask equipped with a reflux tube, and stirred at room temperature under nitrogen flow. 35.6 g (427 mmol) of 48% aqueous sodium hydroxide solution was added, and the temperature was raised to 60°C. Then, 21.7 g (183 mmol) of 3-bromo-1-propyne was added dropwise, and the mixture was stirred at 60°C for 2 hours. The mixture was cooled to 30°C, and the solvent was evaporated off from the organic layer. Then, 95 g of methanol was added, and the precipitate was collected by filtration. The filter cake was washed with methanol/ion-exchange water, and dried under reduced pressure at 40°C to obtain Compound A23 as a pale yellow powder.
Actual yield: 6.9 g (percent yield: 45.0%)
¹H-NMR (DMSO-d₆) δ/ppm: 2.22-2.24 (m, 2H), 2.26-2.28 (m, 2H), 2.35-2.38 (m, 4H), 2.81-2.82 (m, 2H), 2.85-2.87 (m, 2H), 3.58 (t, 1H), 3.65 (t, 2H), 4.87 (d, 2H), 4.97 (d, 4H), 7.07-7.10 (m, 2H), 7.22-7.29 (m, 3H), 7.58-7.63 (m, 5H)

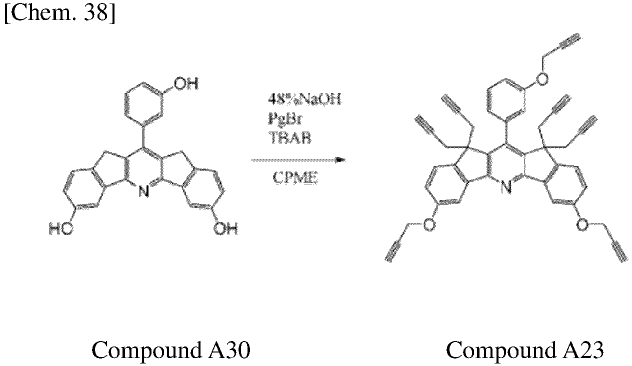

### Example 1-17

### (Step 1)

Compound A31 as a pale yellow powder was obtained in the same synthetic manner as in Example 1-7, except that 1-indanone was changed to 5-hydroxy-1-indanone. ¹H-NMR (DMSO-d₆) δ/ppm: 3.85 (s, 4H), 4.06 (s, 2H), 6.90 (dd, 2H), 7.00 (d, 2H), 7.38 (t, 1H), 7.45 (t, 1H), 7.66 (d, 1H), 7.74 (d, 1H), 7.87 (d, 2H), 7.96 (s, 1H), 8.01 (d, 1H), 8.09 (d, 1H), 9.67 (s, 2H)

### (Step 2)

14 g (31.0 mmol) of compound A31, 3.00 g (9.3 mmol) of tetrabutylammonium bromide, and 56 g of cyclopentyl methyl ether were placed in a reaction flask equipped with a reflux tube, and stirred at room temperature under nitrogen flow. 62.0 g (744 mmol) of 48% aqueous sodium hydroxide solution was added, and the temperature was raised to 60°C. Then, 31.0 g (261 mmol) of 3-bromo-1-propyne was added dropwise, and the mixture was stirred at 60°C for 5 hours. The mixture was cooled to 30°C, and the insoluble matters in the reaction system were removed and the solvent was evaporated off. Then, 60 g of cyclopentyl methyl ether, 120 g of ion-exchange water, and 60 g of methanol were added, and the precipitate was collected by filtration. The filter cake was washed with methanol/ion-exchange water, and dried under reduced pressure 50°C to obtain Compound A24 as a yellow powder.
Actual yield: 15.2 g (percent yield: 64.9%)
¹H-NMR (DMSO-d₆) δ/ppm: 2.48 (dd, 2H), 2.76-2.84 (m, 4H), 2.90-3.00 (m, 4H), 3.62 (dd, 2H), 4.88-4.90 (m, 4H), 7.12 (dd, 2H), 7.31 (d, 2H), 7.41-7.50 (m, 2H), 7.62 (d, 1H), 7.76 (d, 1H), 7.92 (d, 2H), 7.97-7.99 (m, 2H), 8.11 (d, 1H)

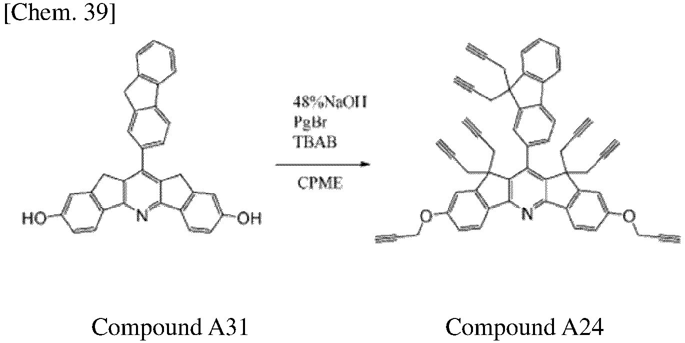

### Example 1-18

### (Step 1)

Compound A32 as a pale yellow powder was obtained in the same synthetic manner as in Example 1-7, except that 1-indanone was changed to 6-hydroxy-1-indanone. ¹H-NMR (DMSO-d₆) δ/ppm: 3.80 (s, 4H), 4.00 (s, 2H), 6.84 (dd, 2H), 7.35-7.39 (m, 3H), 7.44 (t, 1H), 7.48 (d, 2H), 7.66 (d, 1H), 7.74 (d, 1H), 7.99 (d, 2H), 8.06 (d, 1H), 9.53 (s, 2H)

### (Step 2)

60 g (132.9 mmol) of compound A32, 12.85 g (39.9 mmol) of tetrabutylammonium bromide, and 360 g of cyclopentyl methyl ether were placed in a reaction flask equipped with a reflux tube, and stirred at room temperature under nitrogen flow. 265.7 g (3189 mmol) of 48% aqueous sodium hydroxide solution was added, and the temperature was raised to 60°C. Then, 139.1 g (1169 mmol) of 3-bromo-1-propyne was added dropwise, and the mixture was stirred at 60°C for 3 hours. The mixture was cooled to 30°C, and the insoluble matters in the reaction system were removed and the solvent was evaporated off. Then, 60 g of cyclopentyl methyl ether, 120 g of ion-exchange water, and 60 g of methanol were added, and the precipitate was collected by filtration. The filter cake was washed with methanol/ion-exchange water, and dried under reduced pressure 50°C to obtain Compound A25 as a yellow powder.
Actual yield: 19.55 g (percent yield: 19.5%)
¹H-NMR (DMSO-d₆) δ/ppm: 2.28 (dd, 2H), 2.35-2.45 (m, 6H), 2.45 (t, 2H), 2.80 (td, 4H), 2.95 (d, 4H), 3.66 (t, 2H), 4.98 (d, 4H), 7.08 (dd, 2H), 7.42-7.50 (m, 2H), 7.56-7.64 (m, 5H), 7.76 (d, 1H), 7.98 (d, 2H), 8.12 (d, 1H)

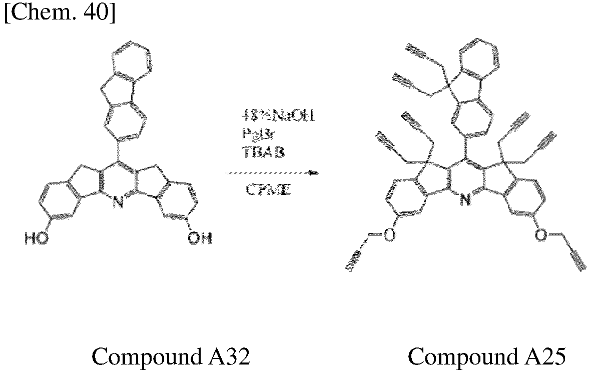

### Example 1-19

### (Step 1)

Compound A33 as a pale yellow powder was obtained in the same synthetic manner as in Example 1-1, except that 4-hydroxybenzaldehyde was changed to 3,4-dihydroxybenzaldehyde.
¹H-NMR (DMSO-d₆) δ/ppm: 3.91 (s, 4H), 6.96 (d, 4H), 7.09 (s, 1H), 7.16 (s, 1H), 7.48 (d, 2H), 7.79 (d, 2H), 9.78 (s, 4H)

### (Step 2)

22.7 g (57.3 mmol) of compound A33, 5.4 g (16.8 mmol) of tetrabutylammonium bromide, and 310 g of tetrahydrofuran were placed in a reaction flask equipped with a reflux tube, and stirred at room temperature under nitrogen flow. 361 g (988 mmol) of 25% aqueous tetramethylammonium hydroxide solution was added, and the temperature was raised to 45°C. Then, 72.3 g (608 mmol) of 3-bromo-1-propyne was added dropwise, and the mixture was stirred at 45 for 2 hours. The mixture was cooled to 30°C, and the solvent was evaporated off from the organic layer. Then, 95 g of methanol was added, and the precipitate was collected by filtration. The filter cake was washed with methanol/ion-exchange water, and dried under reduced pressure at 40°C to obtain Compound A26 as a yellow powder.
Actual yield: 15.1 g (percent yield: 37.3%)
¹H-NMR (DMSO-d₆) δ/ppm: 2.36 (s, 8H), 2.83 (s, 4H), 3.46 (s, 1H), 3.65 (s, 3H), 4.78 (s, 2H), 4.50 (s, 6H), 7.06 (d, 2H), 7.16 (d, 1H), 7.32 (d, 2H), 7.57 (s, 3H), 7.59 (d, 1H)

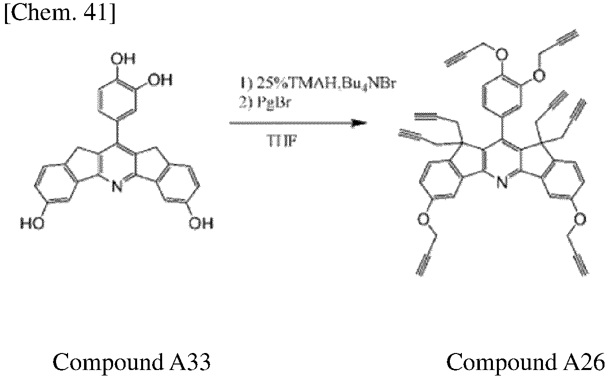

### Example 1-20

### (Step 1)

Compound A34 as a pale yellow powder was obtained in the same synthetic manner as in Example 1-1, except that 4-hydroxybenzaldehyde was changed to 2-hydroxy-1-naphthaldehyde.
¹H-NMR (DMSO-d₆) δ/ppm: 3.32 (s, 4H), 6.82 (dd, 2H), 7.14-7.17 (m, 1H), 7.30-7.35 (m, 4H), 7.39 (d, 1H), 7.51 (d, 2H), 7.91-7.97 (m, 2H), 9.54 (s, 2H), 9.86 (s, 1H)

### (Step 2)

8.0 g (18.6 mmol) of compound A34, 1.8 g (5.6 mmol) of tetrabutylammonium bromide, and 48.0 g of cyclopentyl methyl ether were placed in a reaction flask equipped with a reflux tube, and stirred at room temperature under nitrogen flow. 32.6 g (391 mmol) of 48% aqueous sodium hydroxide solution was added, and the temperature was raised to 60°C. Then, 17.1 g (143 mmol) of 3-bromo-1-propyne was added dropwise, and the mixture was stirred at 60° for 5 hours. The mixture was cooled to 30°C and the solvent was evaporated off from the organic layer. Then, 95 g of methanol was added, and the precipitate was collected by filtration. The filter cake was washed with methanol/ion-exchange water, and dried under reduced pressure at 40°C to obtain Compound A27 as a pale yellow powder.
Actual yield: 2.8 g (percent yield: 21.6%)
¹H-NMR (DMSO-d₆) δ/ppm: 2.22-2.24 (m, 2H), 2.26-2.28 (m, 2H), 2.35-2.38 (m, 4H), 2.81-2.82 (m, 2H), 2.85-2.87 (m, 2H), 3.58 (t, 1H), 3.65 (t, 2H), 4.87 (d, 2H), 4.97 (d, 4H), 7.07-7.10 (m, 2H), 7.22-7.29 (m, 3H), 7.58-7.63 (m, 5H)

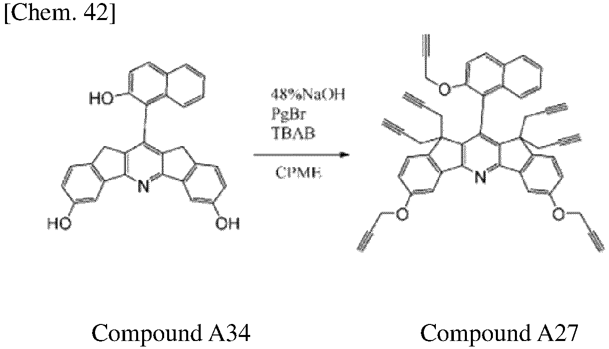

### Example 1-21

### (Step 1)

Compound A35 as a pale yellow powder was obtained in the same synthetic manner as in Example 1-1, except that 4-hydroxybenzaldehyde was changed to 4-acetamidobenzaldehyde.
¹H-NMR (DMSO-d₆) δ/ppm: 2.10 (s, 3H), 3.79 (s, 4H), 6.84 (dd, 2H), 7.37 (d, 2H), 7.45 (d, 2H), 7.67-7.71 (m, 2H), 7.76 (d, 2H), 9.52 (s, 2H), 10.13 (s, 1H)

### (Step 2)

60.0 g (143 mmol) of compound A35, 240 g of N-methyl-1-pyrrolidone, and 240 g of ethanol were placed in a reaction flask equipped with a reflux tube, and stirred at room temperature under nitrogen flow. 179 g (2140 mmol) of 48% aqueous sodium hydroxide solution was added, and the mixture was stirred at 90°C for 10 hours. The mixture was cooled to 30°C, 108 g of ethyl acetate was added, and the precipitate was collected by filtration. The filter cake was washed with ethyl acetate and then with methanol/ion-exchange water, and dried under reduced pressure at 40°C to obtain Compound A36 as a pale yellow powder.
¹H-NMR (DMSO-d₆) δ/ppm: 3.74 (s, 4H), 5.36 (s, 2H), 6.70 (d, 2H), 6.75 (d, 2H), 7.29 (d, 2H), 7.41 (d, 2H), 7.45 (d, 2H)

### (Step 3)

16.0 g (42.3 mmol) of compound A36, 4.1 g (12.7 mmol) of tetrabutylammonium bromide, and 160 g of cyclopentyl methyl ether were placed in a reaction flask equipped with a reflux tube, and stirred at room temperature under nitrogen flow. 52.9 g (634 mmol) of 48% aqueous sodium hydroxide solution was added, and the temperature was raised to 65°C. Then, 44.3 g (372 mmol) of 3-bromo-1-propyne was added dropwise, and the mixture was stirred at 75° for 7 hours. The mixture was cooled to 30°C and the precipitate was collected by filtration. Then, the organic layer of the filtrate was washed with water five times, and dried under reduced pressure at 40°C. The solid obtained was washed with methyl isobutyl ketone, and the filter cake was dried under reduced pressure at 40°C to obtain Compound A28 as a pale yellow powder.
Actual yield: 19.5 g (percent yield: 67.5%)
¹H-NMR (DMSO-d₆) δ/ppm: 1.70 (dd, 2H), 2.25 (dd, 4H), 2.37 (t, 2H), 2.54-2.67 (m, 4H), 3.53 (t, 1H), 3.67 (t, 2H), 4.84 (d, 2H), 4.98 (d, 4H), 7.06 (dd, 2H), 7.30 (t, 1H), 7.47 (t, 1H), 7.59-7.65 (m, 5H), 7.72 (d, 1H), 8.02 (d, 1H), 8.27 (d, 1H)

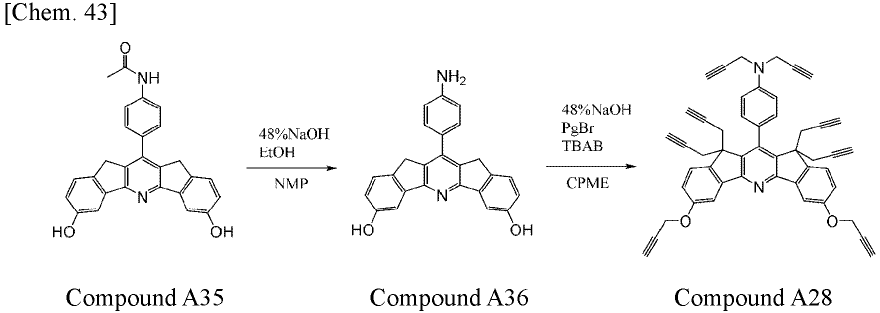

### Examples 2-1 to 2-25 and Comparative Examples 1 to 4

Components were weighed according to the formulation (parts by mass) shown in Tables 1 to 4 and mixed, and then the solids were dissolved by stirring. After confirming that the solids were completely dissolved, the solution was filtered through a fluororesin filter (pore size: 0.2 µm) to obtain a composition for evaluation.

The components shown in Tables were as follows.
Compound A8: Compound produced in Example 1-6
Compound A10: Compound produced in Example 1-7
Compound A11: Compound produced in Example 1-8
Compound A12: Compound produced in Example 1-9
Compound A13: Compound produced in Example 1-10
Compound A14: Compound produced in Example 1-11
Compound A15: Compound produced in Example 1-12
Compound A17: Compound produced in Example 1-13
Compound A18: Compound produced in Example 1-14
Compound A22: Compound produced in Example 1-15
Compound A23: Compound produced in Example 1-16
Compound A24: Compound produced in Example 1-17
Compound A25: Compound produced in Example 1-18
Compound A26: Compound produced in Example 1-19
Compound A27: Compound produced in Example 1-20
Compound A28: Compound produced in Example 1-21
Compound A20: Compound having the structure below, which has no reactive group
Compound A21: Compound having the structure below, which has reactive groups but is different from the compound I

Compound B1: Cross-linking agent having the structure below (glycoluril compound)
Compound B2: Cross-linking agent having the structure below (phenol compound)
Compound C1: Bis(4-tert-butylphenyl)iodonium nonafluorobutanesulfonate (polymerization initiator: thermal acid generator)
Compound D1: Propylene glycol monomethyl ether acetate (PGMEA), solvent
Compound D2: Cyclohexanone, solvent

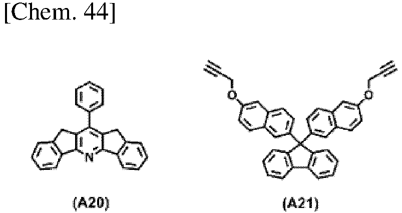

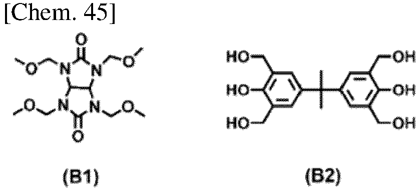

### Preparation of Substrate for Evaluation

The composition for evaluation were applied to a silicon wafer substrate using a spin coater so as to achieve a film thickness of 200 nm after heating. The coated substrate was heated on a hot plate at 170°C for 60 seconds and then further heated on a hot plate at 300°C for 60 seconds, to obtain a substrate for evaluation.

The substrate for evaluation was used to evaluate various characteristics in the following manner. Tables 1 to 4 collectively show the evaluation results.

### Film-Forming Property

In the preparation of the substrate for evaluation mentioned above, the film thickness after heating on a hot plate at 170°C for 60 seconds (W1) and the film thickness after heating on a hot plate at 300°C for another 60 seconds (W2) were measured. Also, the film surface on the substrate for evaluation was visually observed.

If the film had a uniform surface with no discoloration and also with a rate of change in the film thickness ((W1-W2)/W1) less than 5%, the composition was graded A+. If the film had a uniform surface with no discoloration and also with a rate of change in the film thickness of 5% or more and less than 10%, the composition was graded A. If the film had a uniform surface with discoloration (e.g., yellowing or blackening), the composition was graded B. If the film had a non-uniform surface due to, for example, precipitation or volatilization and also had discoloration, the composition was graded C.

It can be concluded that a composition is excellent in the film-forming property if the film had a uniform surface with no discoloration and also with a small rate of change in the film thickness.

### Heat Resistance

The film thickness of the substrate for evaluation was measured at five points using a spectroscopic ellipsometer SE-2000 manufactured by Semilab Inc., and the average value thereof was used as the film thickness before the test.

The substrate for evaluation was heated at 300°C for 60 seconds, and the film thickness after heating was measured in the same manner to obtain the film thickness after the test (Wa).

If the rate of the change from the film thickness before the test to that after the test (Wb-Wa), relative to the film thickness before the test (Wb), was less than 3%, the composition was graded A. If the rate was 3% or more and less than 5%, the composition was graded B. If the rate was 5% or more and less than 10%, the composition was graded C. If the rate was 10% or more, the composition was graded D.

Among Examples in which the grade B was given, the rates of the change in the film thickness in Examples 2-3, 2-5, 2-6, 2-7, 2-13, 2-14, 2-18, 2-19, and 2-22 were lower than that in Example 2-4, though not shown as data.

### Solvent Resistance

The film thickness of the substrate for evaluation was measured at five points using a spectroscopic ellipsometer SE-2000 manufactured by Semilab Inc., and the average value thereof was used as the film thickness before the test.

The substrate for evaluation was immersed in PGMEA at 25°C for 60 seconds and then heated at 170°C for 60 seconds to evaporate the PGMEA, and the film thickness was measured in the same manner to obtain the film thickness after the test (Wa').

If the rate of the change from the film thickness before the test to that after the test (Wb'-Wa'), with respect to the film thickness before the test (Wb'), was less than 1%, the composition was graded A. If the rate was 1% or more and less than 3%, the composition was graded B. If the rate was 3% or more, the composition was graded C.

### Filling Property and Planarization

The composition was applied to a SiO₂ stepped substrate (wall: 500 nm wide and 100 nm high; trench: 500 nm wide) using a spin coater. The resulting substrate was heated at 170°C for 60 seconds and 300°C for 60 seconds to form a film. The spin-coating conditions were adjusted so that the film thickness was 200 nm from the trench.

Evaluation of Filling Property: A cut piece of the substrate was prepared, and observed using a SEM (S-4800 manufactured by Hitachi High-Tech Corporation). If the step was filled without voids, the composition was graded A; and if the step was filled with voids, the composition was graded B.

Evaluation of Planarization: The planarization was evaluated on the basis of the difference (difference in the film thickness) between the level at the thickest point of the film on the substrate wall and the level at the thinnest point of the film on the trench. If the difference in the film thickness was less than 10 nm, the composition was graded A; if the difference was 10 nm or more and less than 30 nm, the composition was graded B, and if the difference was 30 nm or more, the composition was graded C.

The smaller the difference in film thickness, the higher the planarization, and such a composition can be more suitably used as a material for an electronic component, especially as a material for forming film in semiconductor processing.

**Table 1**

| | | Examples | | | | | | | | |
|---|---|---|---|---|---|---|---|---|---|---|
| | Compound | 2-1 | 2-2 | 2-3 | 2-4 | 2-5 | 2-6 | 2-7 | 2-8 | 2-9 |
| | A8 | 5 | | | | | | | | |
| | A10 | | 5 | | | | | | | |
| | A11 | | | 5 | | | | | | |
| | A12 | | | | 5 | | | | | |
| | A13 | | | | | 5 | | | | |
| | A14 | | | | | | 5 | | | |
| | A15 | | | | | | | 5 | | |
| | A17 | | | | | | | | 5 | |
| | A18 | | | | | | | | | 5 |
| | A20 | | | | | | | | | |
| Composition | A21 | | | | | | | | | |
| | A22 | | | | | | | | | |
| | A23 | | | | | | | | | |
| | A24 | | | | | | | | | |
| | A25 | | | | | | | | | |
| | A26 | | | | | | | | | |
| | A27 | | | | | | | | | |
| | A28 | | | | | | | | | |
| | B1 | | | | | | | | | |
| | B2 | | | | | | | | | |
| | C1 | | | | | | | | | |
| | D1 | 47.5 | 47.5 | 47.5 | 47.5 | 47.5 | 47.5 | 47.5 | 47.5 | 47.5 |
| | D2 | 47.5 | 47.5 | 47.5 | 47.5 | 47.5 | 47.5 | 47.5 | 47.5 | 47.5 |
| | | | | | | | | | | |
| Evaluation | Film-forming property | A | A | A+ | A | A+ | A | A | A | A+ |
| | Heat resistance | A | A | B | B | B | B | B | A | A |
| | Solvent resistance | A | A | A | A | A | A | A | A | A |
| | Filling property | A | A | A | A | A | A | A | A | A |
| | Planarization | A | A | A | A | A | A | A | A | A |

**Table 2**

| | | Examples | | | | | | | |
|---|---|---|---|---|---|---|---|---|---|
| | Compound | 2-10 | 2-11 | 2-12 | 2-13 | 2-14 | 2-15 | 2-16 | 2-17 |
| | A8 | | | | | | | | |
| | A10 | 2.5 | 3.5 | 4 | | | | | |
| | A11 | 2.5 | | | 4 | | | | |
| | A12 | | | | | | | | |
| | A13 | | | | | 4 | | | |
| | A14 | | | | | | | | |
| | A15 | | | | | | | | |
| | A17 | | | | | | | | |
| | A18 | | | | | | 4 | 4 | 4.5 |
| | A20 | | | | | | | | |
| Composition | A21 | | 1.5 | | | | | | |
| | A22 | | | | | | | | |
| | A23 | | | | | | | | |
| | A24 | | | | | | | | |
| | A25 | | | | | | | | |
| | A26 | | | | | | | | |
| | A27 | | | | | | | | |
| | A28 | | | | | | | | |
| | B1 | | | 0.5 | 0.5 | 0.5 | 0.5 | | |
| | B2 | | | | | | | 0.5 | |
| | C1 | | | 0.5 | 0.5 | 0.5 | 0.5 | 0.5 | 0.5 |
| | D1 | 47.5 | 47.5 | 47.5 | 47.5 | 47.5 | 47.5 | 47.5 | 47.5 |
| | D2 | 47.5 | 47.5 | 47.5 | 47.5 | 47.5 | 47.5 | 47.5 | 47.5 |
| | | | | | | | | | |
| Evaluation | Film-forming property | A | A | A | A | A | A | A | A |
| | Heat resistance | A | A | A | B | B | A | A | A |
| | Solvent resistance | A | A | A | A | A | A | A | A |
| | Filling property | A | A | A | A | A | A | A | A |
| | Planarization | A | A | A | A | A | A | A | A |

**Table 3**

| | | Examples | | | | | | |
|---|---|---|---|---|---|---|---|---|
| | Compound | 2-18 | 2-19 | 2-20 | 2-21 | 2-22 | 2-23 | 2-24 |
| | A8 | | | | | | | |
| | A10 | | | | | | | |
| | A11 | | | | | | | |
| | A12 | | | | | | | |
| | A13 | | | | | | | |
| | A14 | | | | | | | |
| | A15 | | | | | | | |
| | A17 | | | | | | | |
| | A18 | | | | | | | |
| | A20 | | | | | | | |
| Composition | A21 | | | | | | | |
| | A22 | 5 | | | | | | |
| | A23 | | 5 | | | | | |
| | A24 | | | 5 | | | | |
| | A25 | | | | 5 | | | |
| | A26 | | | | | 5 | | |
| | A27 | | | | | | 5 | |
| | A28 | | | | | | | 5 |
| | B1 | | | | | | | |
| | B2 | | | | | | | |
| | C1 | | | | | | | |
| | D1 | 47.5 | 47.5 | 47.5 | 47.5 | 47.5 | 47.5 | 47.5 |
| | D2 | 47.5 | 47.5 | 47.5 | 47.5 | 47.5 | 47.5 | 47.5 |
| | | | | | | | | |
| Evaluation | Film-forming property | A+ | A+ | A+ | A+ | A+ | A+ | A+ |
| | Heat resistance | B | B | A | A | B | A | A |
| | Solvent resistance | A | A | A | A | A | A | A |
| | Filling property | A | A | A | A | A | A | A |
| | Planarization | A | A | A | A | A | A | A |

**Table 4**

| | | Comparative Examples | | | |
|---|---|---|---|---|---|
| | Compound | 1 | 2 | 3 | 4 |
| | A8 | | | | |
| | A10 | | | | |
| | A11 | | | | |
| | A12 | | | | |
| | A13 | | | | |
| | A14 | | | | |
| | A15 | | | | |
| | A17 | | | | |
| | A18 | | | | |
| | A20 | 5 | 4 | | |
| Composition | A21 | | | 5 | 4 |
| | A22 | | | | |
| | A23 | | | | |
| | A24 | | | | |
| | A25 | | | | |
| | A26 | | | | |
| | A27 | | | | |
| | A28 | | | | |
| | B1 | | 0.5 | | 0.5 |
| | B2 | | | | |
| | C1 | | 0.5 | | 0.5 |
| | D1 | 47.5 | 47.5 | 47.5 | 47.5 |
| | D2 | 47.5 | 47.5 | 47.5 | 47.5 |
| | | | | | |
| Evaluation | Film-forming property | C | C | B | B |
| | Heat resistance | D | D | C | C |
| | Solvent resistance | C | C | B | B |
| | Filling property | B | B | B | B |
| | Planarization | B | B | B | B |

As shown in Tables 1 to 3, films excellent in heat resistance and solvent resistance was obtained by using the compound I of the present invention. In addition, the compound of the present invention was excellent in the film-forming property, and also the filling property and planarization during film formation.

On the other hand, it was found that films obtained by using compounds that did not fall under the formula (I) were inferior in heat resistance or inferior solvent resistance, as shown in Table 4.

## Claims

1. A compound represented by a general formula (I) below: 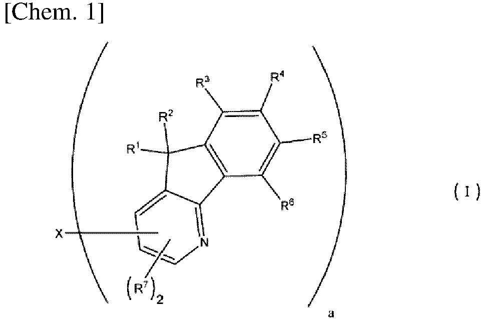
where X represents a hydrogen atom, an optionally substituted ring group having 2 to 20 ring-constituting carbon atoms, an optionally substituted chain hydrocarbon group having 1 to 20 carbon atoms, or a group obtained by replacing one or more methylene groups in the chain hydrocarbon group with a divalent group selected from <Group B> below;
R¹, R², R³, R⁴, R⁵, R⁶, and R⁷ each independently represent a hydrogen atom, a reactive group selected from <Group A> below, a nitro group, an optionally substituted alkyl group having 1 to 20 carbon atoms, an optionally substituted aromatic ring group having 6 to 20 carbon atoms, an optionally substituted heterocyclic group having 2 to 20 carbon atoms, a group obtained by replacing one or more methylene groups in the alkyl group having 1 to 20 carbon atoms with a divalent group selected from <Group B> below, a group obtained by replacing one or more methylene groups in the aromatic ring group having 6 to 20 carbon atoms with a divalent group selected from <Group B> below, or a group obtained by replacing one or more methylene groups in the heterocyclic group having 2 to 20 carbon atoms with a divalent group selected from <Group B> below;
<Group A> consists of a carbon-carbon unsaturated bond-containing group, a cyclic ether group, a hydroxyl group, a mercapto group, an amino group, a halogen atom, and a cyano group,
<Group B> consists of -O-, -CO-, -COO-, -OCO-, -NR¹¹-, -NR¹¹CO-, and -S-, where R¹¹ represents a hydrogen atom or a hydrocarbon group;
two R⁷ in one repeating unit are the same or different;
R¹ and R² are optionally bonded together to form a ring,
R³ and R⁴ are optionally bonded together to form a ring,
R⁴ and R⁵ are optionally bonded together to form a ring,
R⁵ and R⁶ are optionally bonded together to form a ring,
a plurality of R⁷ are optionally bonded together to form a ring;
*a* represents an integer of 1 to 10, and
in a plurality of repeating units when *a* is 2 or more, a plurality of R¹ are the same or different, a plurality of R² are the same or different, a plurality of R³ are the same or different, a plurality of R⁴ are the same or different, a plurality of R⁵ are the same or different, a plurality of R⁶ are the same or different, and a plurality of R⁷ are the same or different;
provided that the compound has one or more reactive groups selected from <Group A> in a molecule thereof.

2. The compound according to claim 1, represented by a general formula (IIα), (IIβ), or (IIγ) below: 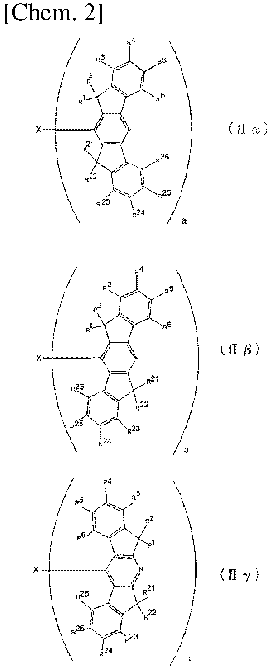
where X, *a,* R¹, R², R³, R⁴, R⁵, and R⁶ are as in the general formula (I);
R²¹, R²², R²³, R²⁴, R²⁵, and R²⁶ each independently represent a hydrogen atom, a reactive group selected from <Group A> above, a nitro group, an optionally substituted alkyl group having 1 to 20 carbon atoms, an optionally substituted aromatic ring group having 6 to 20 carbon atoms, an optionally substituted heterocyclic group having 2 to 20 carbon atoms, a group obtained by replacing one or more methylene groups in the alkyl group having 1 to 20 carbon atoms with a divalent group selected from <Group B> above, a group obtained by replacing one or more methylene groups in the aromatic ring group having 6 to 20 carbon atoms with a divalent group selected from <Group B> above, or a group obtained by replacing one or more methylene groups in the heterocyclic group having 2 to 20 carbon atoms with a divalent group selected from <Group B> above;
R²¹ and R²² are optionally bonded together to form a ring,
R²³ and R²⁴ are optionally bonded together to form a ring,
R²⁴ and R²⁵ are optionally bonded together to form a ring, and
R²⁵ and R²⁶ are optionally bonded together to form a ring.

3. The compound according to claim 1 or 2, wherein X is an optionally substituted aromatic ring group having 6 to 20 ring-constituting carbon atoms or an optionally substituted heterocyclic group having 2 to 20 ring-constituting carbon atoms.

4. The compound according to claim 1, comprising, in the molecule, one or more carbon-carbon unsaturated bond-containing groups as the reactive group.

5. The compound according to claim 2, comprising, in the molecule, one or more carbon-carbon unsaturated bond-containing groups as the reactive group.

6. The compound according to claim 1 or 2, wherein the number of the reactive groups contained in the molecule is two or more.

7. The compound according to claim 1 or 2, wherein X has an optionally substituted fused ring, or the number of the reactive groups contained in the molecule is three or more.

8. A composition comprising the compound according to claim 1 or 2.

9. The composition according to claim 8, being for an electronic component.

10. A cured product from the composition according to claim 8.

11. A method for producing a cured product, comprising the step of curing the composition according to claim 8.

12. A method for producing an electronic component, comprising the step of curing the composition according to claim 8 and then the step of removing the cured product.

13. A compound represented by a general formula (III) below: 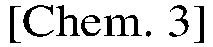 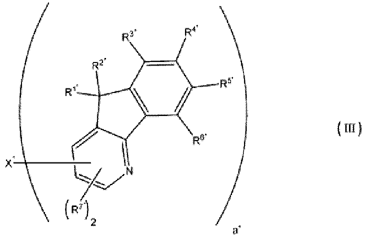
where X' represents a hydrogen atom, an optionally substituted ring group having 2 to 20 ring-constituting carbon atoms, an optionally substituted chain hydrocarbon group having 1 to 20 carbon atoms, or a group obtained by replacing one or more methylene groups in the chain hydrocarbon group with a divalent group selected from <Group B> below;
R¹', R²', R³', R⁴', R⁵', R⁶', and R⁷' each independently represent a hydrogen atom, a reactive group selected from <Group A> below, a nitro group, an optionally substituted alkyl group having 1 to 20 carbon atoms, an optionally substituted aromatic ring group having 6 to 20 carbon atoms, an optionally substituted heterocyclic group having 2 to 20 carbon atoms, a group obtained by replacing one or more methylene groups in the alkyl group having 1 to 20 carbon atoms with a divalent group selected from <Group B> below, a group obtained by replacing one or more methylene groups in the aromatic ring group having 6 to 20 carbon atoms with a divalent group selected from <Group B> below, or a group obtained by replacing one or more methylene groups in the heterocyclic group having 2 to 20 carbon atoms with a divalent group selected from <Group B> below;
<Group A> consists of a carbon-carbon unsaturated bond-containing group, a cyclic ether group, a hydroxyl group, a mercapto group, an amino group, a halogen atom, and a cyano group,
<Group B> consists of -O-, -CO-, -COO-, -OCO-, -NR¹¹'-, -NR¹¹'CO-, and -S-, where R¹¹' represents a hydrogen atom or a hydrocarbon group;
two R⁷' in one repeating unit are the same or different;
R¹' and R²' are optionally bonded together to form a ring,
R³' and R⁴' are optionally bonded together to form a ring,
R⁴' and R⁵' are optionally bonded together to form a ring,
R⁵' and R⁶' are optionally bonded together to form a ring,
a plurality of R⁷' are optionally bonded together to form a ring;
*a*' represents an integer of 1 to 10,
when X' is a hydrogen atom, *a*' is 1, and
in a plurality of repeating units when *a*' is 2 or more, a plurality of R¹' are the same or different, a plurality of R²' are the same or different, a plurality of R³' are the same or different, a plurality of R⁴' are the same or different, a plurality of R⁵' are the same or different, a plurality of R⁶' are the same or different, and a plurality of R⁷' are be the same or different;
provided that the compound has one or more phenolic hydroxyl groups in a molecule thereof.
